(19) Europäisches Patentamt
European Patent Office
Office européen des brevets

(11) Veröffentlichungsnummer : **0 044 928 B1**

(12) **EUROPÄISCHE PATENTSCHRIFT**

(45) Veröffentlichungstag der Patentschrift : **13.03.85**

(21) Anmeldenummer : **81104476.7**

(22) Anmeldetag : **11.06.81**

(51) Int. Cl.[4] : **C 07 D519/00**, C 07 D493/04, A 61 K 31/52 // (C07D493/04, 307/00, 307/00),(C07D519/00, 493/00, 473/00)

(54) **Durch Purinbasen substituierte 1.4;3.6-Dianhydro-hexit-nitrate, Verfahren zu ihrer Herstellung und pharmazeutische Zubereitung.**

(30) Priorität : **25.07.80 DE 3028273**

(43) Veröffentlichungstag der Anmeldung : **03.02.82 Patentblatt 82/05**

(45) Bekanntmachung des Hinweises auf die Patenterteilung : **13.03.85 Patentblatt 85/11**

(84) Benannte Vertragsstaaten : **AT BE CH FR GB IT LI NL SE**

(56) Entgegenhaltungen :
**US-A- 3 886 186**
**US-A- 4 065 488**
**EUROPEAN JOURNAL OF MEDICINAL CHEMISTRY, CHIMICA THERAPEUTICA, Band 15, Heft 1, Januar 1980, PARIS (FR) G. GIOVANNINETTI et al.: "Synthèse et activité hypocholestérolémique de bis-homoanalogues des nucléosides puriques, Seiten 23-27**
**CHEMICAL ABSTRACTS, Band 92, 1980, Seite 627, Zusammenfassung 147106x, COLUMBUS, OHIO (US) P. ANGIBEAUD et al.: "Homoanalogs of nucleosides"**
**CHEMICAL ABSTRACTS, Band 87, 1977, Seite 513, Zusammenfassung 6280j, COLUMBUS, OHIO (US) Y. ISHIDO et al.: "Synthetic studies by the use of carbonates. Part IX: Reactions of D-mannitol carbonate derivatives catalyzed by tetrathylammonium bromide"**
**CHEMICAL ABSTRACTS, Band 79, 1973, Zusammenfassung 42792w, COLUMBUS, OHIO (US) G. GIOVANNINETTI et al.: "Homoanalogs of aldofuranosyl nucleosides"**

(73) Patentinhaber : **Dr. Willmar Schwabe GmbH & Co.**
**Willmar-Schwabe-Strasse 4**
**D-7500 Karlsruhe 41 (DE)**

(72) Erfinder : **Klessing, Klaus, Dr. Dipl.-Chem.**
**Rheingoldstrasse 3**
**D-7505 Ettlingen 5 (DE)**
Erfinder : **Chatterjee, Shyam Sunder, Dr. Dipl.-Chem.**
**Werrabronner Strasse 8a**
**D-7500 Karlsruhe 41 (DE)**

(74) Vertreter : **Bunke, Holger, Dr. Dipl.-Chem. et al**
**Patentanwälte Prinz, Bunke & Partner Lessingstrasse 9**
**D-7000 Stuttgart 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Erfindung betrifft durch Purinbasen substituierte 1.4;3.6-Dianhydro-hexit-nitrate, nämlich Adenyl-desoxy-1.4;3.6-dianhydro-hexit-nitrate der allgemeinen Formel Ia,

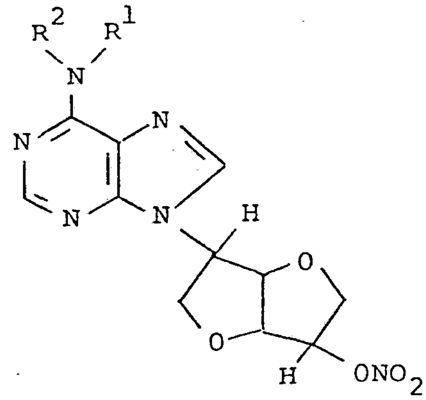

(Ia)

sowie Theophyllinyl-desoxy-1.4;3.6-dianhydro-hexit-nitrate der allgemeinen Formel Ib

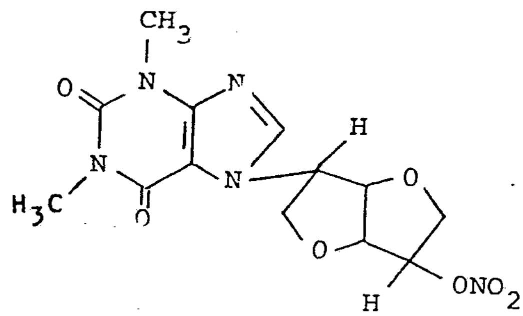

(Ib)

Das Grundgerüst dieser Verbindungen besteht aus einem der stereoisomeren, unter Epimerisierung ineinander umwandelbaren 1.4;3.6-Dianhydro-hexite, nämlich entweder dem 1.4;3.6-Dianhydro-L-idit (= « Isoidid ») (II),

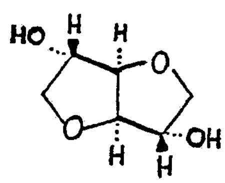

(II)

bei dem die OH-Gruppen in 2- und 5-Stellung jeweils exo-Konfiguration aufweisen,
oder dem 1.4;3.6-Dianhydro-D-glucit (= « Isosorbid ») (III),

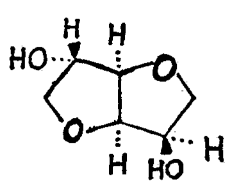

(III)

der eine exo-ständige und eine endo-ständige OH-Gruppe aufweist und somit — bei verschiedenen Substituenten in 2- und 5-Stellung — in zwei isomeren Formen auftritt.

Das Grundgerüst einiger Zwischenprodukte schließlich besteht aus dem 1.4;3.6-Dianhydro-D-mannit (= « Isomannid ») (IV),

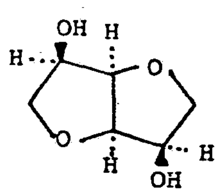

(IV)

der zwei endo-ständige OH-Gruppen aufweist.

Da im Gegensatz zu den Glucit-Derivaten bei den Idit- und Mannit-Derivaten eine Unterscheidung zwischen den 2- und 5-Substituenten nicht möglich ist, weil das $C^2$-Atom bei Drehung des Moleküls um 180° zum $C^5$-Atom wird, sind Hinweise auf die 5-Stellung oder 2-Stellung von Substituenten bei diesen Derivaten an sich überflüssig. Des besseren Vergleiches der Strukturen der einzelnen Verbindungen mit den allgemeinen Formeln wegen werden aber hier die Isoidid-Derivate durchweg als 5-Purinyl-isoidid-Derivate bezeichnet, da sie aus den in 5-Stellung acylsubstituierten Isosorbid-Derivaten entstehen. Entsprechend werden die als Ausgangsverbindungen verwendeten Isomannid-Acylderivate als 2-Acylisomannid-Derivate bezeichnet, da aus ihnen die 2-Purinyl-isosorbid-Derivate hergestellt werden.

Eine kurze Zusammenfassung über die Stereoisomerie der 1.4;3.6-Dianhydro-hexite gibt J.A. Mills in Advances in Carbohydrate Chem. *10,* 1-53 (1955).

Die Erfindung betrifft weiter Verfahren zur Herstellung der eingangs genannten, durch Purinbasen substituierten 1.4;3.6-Dianhydro-hexit-mononitrate sowie pharmazeutische Zubereitungen, die die erfindungsgemäßen Verbindungen enthalten.

Die Nitrate des 1.4;3.6-Dianhydro-D-glucits (auch 1.4;3.6-Dianhydro-D-sorbit genannt) sind z. B. aus der US-A-3 886 186 bekannt, und zwar sowohl die 2- und 5-Mononitrate als auch das 2,5-Dinitrat des Isosorbids. Diese Nitrate, insbesondere das Dinitrat, das bereits als Arzneimittel im Handel ist, sind pharmakologisch wirksame Substanzen mit hämodynamischer, vasodilatorischer und antianginöser Wirksamkeit, die insbesondere bei Koronarinsuffizienz und zur Behandlung von Angina Pectoris verwendet werden.

Aus der US-A-4 065 488 ist ein Verfahren zur Herstellung von Isosorbid-mononitrat durch Acetylierung von Isosorbid mit Essigsäureanhydrid, nachfolgende Nitrierung und anschließende hydrolytische Abspaltung des 2- bzw. 5-Acetylrests bekannt. Durch Purinbasen substituierte 1.4;3.6-Dianhydro-hexit-nitrate und Verfahren zu deren Herstellung sind dagegen nicht beschrieben.

Aus Eur. J. Med. Chem. *15,* 23 (1980) sind durch Purinbasen substituierte 1.4- oder 3.6-Monoanhydrohexit-derivate, jedoch keine Nitrate, und deren Herstellung bekannt. Diese Verbindungen weisen den Cholesterinspiegel senkende Aktivität auf.

Vergleichbare, durch Purinbasen substituierte Monohexit-derivate mit freien Hydroxylgruppen sind auch aus Chemical Abstracts *79,* 42792w (1973) ; *87,* 6280j (1977) ; und *92,* 147106x (1980) bekannt.

Irgendwelche Hinweise auf die erfindungsgemäß beanspruchten Verbindungen und die Verfahren zu ihrer Herstellung lassen sich diesen Druckschriften nicht entnehmen.

Die Pharmakokinetik des Dinitrats und der Mononitrate von Isosorbid, Isomannid und Isoidid wurde von Bogaert und Rosseel in Naunyn-Schmiedeberg's Arch. Pharmacol. *275,* 339 (1972) beschrieben.

Es hat sich jedoch gezeigt, daß die Nitrate unangenehme Nebenwirkungen, insbesondere Kopfschmerzen, verursachen. Die Mononitrate sind außerdem schlechter resorbierbar als etwa das Isosorbiddinitrat (ISDN). Es kommt hinzu, daß sich die Dinitrate des Isosorbids, Isomannids und Isoidids nur unter besonderen Vorsichtsmaßnahmen herstellen und handhaben lassen, da sie explosiv sind.

Somit bestand ein Bedürfnis nach der Bereitstellung neuer pharmazeutischer Mittel mit gleichem Wirkungsspektrum, die jedoch die genannten Nachteile nicht aufweisen, und nach der Schaffung neuer 1.4;3.6-Dianhydro-hexit-mononitrate, die als wirksame Bestandteile solcher pharmazeutischer Mittel verwendet werden können.

Die der Erfindung zugrundeliegende Aufgabe besteht darin, das angeführte Bedürfnis zu befriedigen, die Lösung dieser Aufgabe darin, die erfindungsgemäßen Stoffe bereitzustellen.

Gegenstand der Erfindung sind demnach

1. 5-(9-Adenyl)-5-desoxy-1.4;3.6-dianhydro-L-idit-2-nitrate (=2-(9-Adenyl)-2-desoxy-1.4;3.6-dianhydro-L-idit-5-nitrate) der allgemeinen Formel Ic,

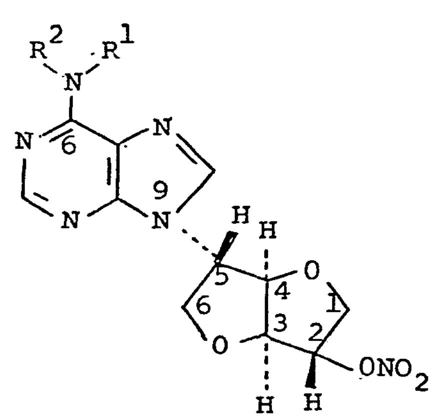

(Ic)

worin $R^1$ und $R^2$ gleich oder verschieden sind und unabhängig voneinander

(a) ein Wasserstoffatom,

(b) eine geradkettige oder verzweigte Alkylgruppe mit 1-7 C-Atomen,

(c) eine ω-Phenylalkylgruppe, wobei die Alkylgruppe 1 bis 7 C-Atome aufweist und wobei der Phenylring in p-Stellung halogensubstituiert sein kann,
bedeuten oder worin

(d) $R^1$ einen der unter (a) bis (c) angegebenen Reste und $R^2$ einen Acylrest einer aliphatischen, gegebenenfalls methylsubstituierten Monocarbonsäure mit 2 bis 7 C-Atomen
bedeuten oder worin

(e) $R^1$ und $R^2$ gemeinsam mit dem Stickstoffatom, an das sie gebunden sind, den Rest eines cyclischen, nicht-aromatischen, gegebenenfalls ein weiteres Heteroatom enthaltenden sekundären Amins darstellen, das ausgewählt ist aus der aus Pyrrolidin, Piperidin, Piperazin, 4-Methylpiperazin, Morpholin bestehenden Gruppe, sowie deren physiologisch unbedenkliche Säureadditionssalze.

2. 2-(9-Adenyl)-2-desoxy-1.4;3.6-dianhydro-D-glucit-5-nitrate der allgemeinen Formel Id,

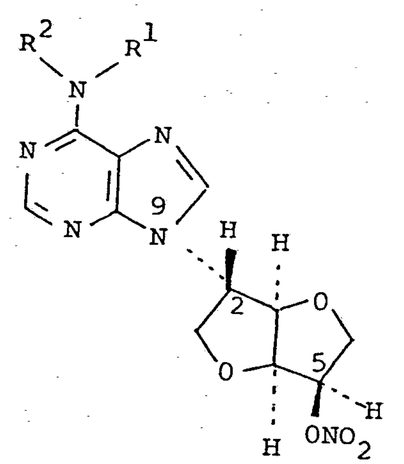

(Id)

worin $R^1$ und $R^2$ die unter (1.) genannten Bedeutungen besitzen, sowie deren physiologisch unbedenkliche Säureadditionssalze.

3. 5-(9-Adenyl)-5-desoxy-1.4;3.6-dianhydro-D-glucit-2-nitrate der allgemeinen Formel Ie,

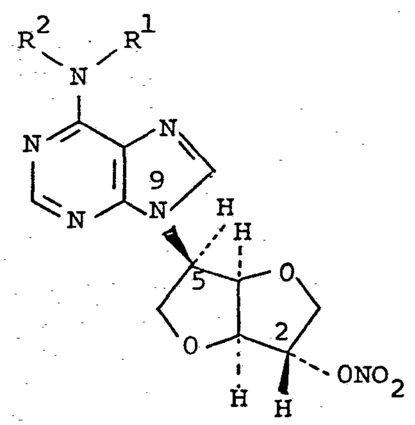

(Ie)

worin $R^1$ und $R^2$ die unter (1.) genannten Bedeutungen besitzen, sowie deren physiologisch unbedenkliche Säureadditionssalze.

4. 5-(7-Theophyllinyl)-5-desoxy-1.4;3.6-dianhydro-L-idit-2-nitrat (= 2-(7-Theophyllinyl)-2-desoxy-1.4;3.6-dianhydro-L-idit-5-nitrat) sowie dessen physiologisch unbedenkliche Säureadditionssalze.

5. 2-(7-Theophyllinyl)-2-desoxy-1.4;3.6-dianhydro-D-glucit-5-nitrat sowie dessen physiologisch unbedenkliche Säureadditionssalze.

Die erfindungsgemäßen Verbindungen besitzen koronardurchflußsteigernde, spasmolytische und blutdrucksenkende Wirksamkeit. Sie sind zur Behandlung von Koronarerkrankungen, zur Kupierung und Prophylaxe von Angina Pectoris-Anfällen, zur Nachbehandlung von Herzinfarkten und zur Behandlung von Herzinsuffizienzen geeignet. Die neuen Verbindungen besitzen eine gute therapeutische Breite. Die orale Absorption ist besonders gut und die Wirkungsdauer lang. Darüber hinaus bewirken sie eine Verbesserung der peripheren Durchblutung und der Gehirndurchblutung.

Die Handhabung und Herstellung der erfindungsgemäßen Verbindungen ist weit weniger gefährlich als etwa beim bekannten ISDN, weil sie nicht explosiv sind.

Die erfindungsgemäßen Verbindungen besitzen im 1.4;3.6-Dianhydro-hexit-Grundgerüst 4 asymmetrische C-Atome und liegen in optisch aktiver Form vor, da als Ausgangsprodukte optisch reine 1.4;3.6-Dianhydro-hexite verwendet werden, die aus natürlich vorkommenden Zuckeralkoholen leicht zugänglich sind.

Die erfindungsgemäßen Verbindungen können ausgehend von den epimeren, unsubstituierten 1.4;3.6-Dianhydro-hexiten, also ausgehend von L-Isoidid, D-Isosorbid und D-Isomannid, hergestellt werden, wobei im Falle des D-Isosorbids als Ausgangsverbindung zwei verschiedene Synthesewege möglich sind.

Der erste Weg besteht erfindungsgemäß darin, daß der entsprechende 1.4;3.6-Dianhydro-hexit mit einem Sulfonsäurechlorid, vorzugsweise mit Methansulfonsäurechlorid oder Toluolsulfonsäurechlorid, in einem geeigneten wasserfreien Lösungsmittel und in Gegenwart einer Hilfsbase, vorzugsweise in Pyridin oder in Chloroform/Triäthylamin, bei erniedrigter Temperatur, vorzugsweise zwischen − 20° und + 10 °C, in den entsprechenden mono-O-acyl-1.4;3.6-dianhydro-hexit übergeführt und das Acyl-Derivat mit dem Alkalisalz, meist dem Natrium- oder Kalium-Salz des gewünschten Purin-Derivats, vorzugsweise des Adenins, 6-N-substituierten Adenins, Theophyllins, 6-Methylmercaptopurins oder des 6-Chlorpurins, in einem dipolaren aprotischen Lösungsmittel, z. B. Dimethylformamid, Dimethylsulfoxid, Tetramethylharnstoff, Diäthern des Äthylenglykols, vorzugsweise in Dimethylformamid oder Dimethylsulfoxid, bei erhöhter Temperatur, vorzugsweise bei 80 bis 130 °C, unter Inertgasatmosphäre ($N_2$ oder Ar) umgesetzt wird.

Der in dieser ersten Stufe erfolgende Austausch der Mesylat- bzw. Tosylatgruppe durch den Adenyl-, 6-N-substituierten Adenyl-, Theophyllinyl-, 6-Chlorpurinyl- oder 6-Methylmercaptopurinyl-Rest verläuft nach dem Reaktionsmuster einer typischen bimolekularen nucleophilen Substitution ($S_N2$-Reaktion), die stets mit einer Umkehr der Konfiguration am zentralen Kohlenstoffatom verbunden ist. Diese Konfigurationsumkehr, dem Fachmann auch unter den Begriffen « Inversion » oder « Walden-Umkehr » geläufig, ist der Grund dafür, daß aus dem 1.4;3.6-Dianhydro-D-glucit-5-acyl-Derivat, bei dem der Acylrest in 5-Stellung endo-ständig vorliegt, stets das in 5-Stellung durch den Adenyl-, 6-N-substituierten Adenyl-, Theophyllinyl-, 6-Chlorpurinyl- oder 6-Methylmercaptopurinyl-Rest substituierte 1.4;3.6-Dianhydro-L-idit-Derivat entsteht, bei dem der anstelle des Acyl-Rests in das Molekül eingetretene Substituent nicht mehr in endo-Stellung, sondern in exo-Stellung steht. Die mit der $S_N2$-Reaktion verbundene Walden-Umkehr ist in ganz entsprechender Weise verantwortlich dafür, daß aus dem entsprechenden Idit-Acylat stets das in 5-Stellung endo-substituierte Glucit-Derivat und aus dem Mannit-Acylat das entsprechende, in 2-Stellung exo-substituierte Glucit-Derivat entsteht.

Ganz analog müßte also aus dem 1.4;3.6-Dianhydro-D-glucit-2-acylat, bei dem sich der Acylrest in exo-Stellung befindet, das entsprechende 2-Purinyl-1.4;3.6-djanhydro-D-mannit-Derivat entstehen, bei dem der Purinrest dann endo-ständig sein müßte. Es hat sich jedoch gezeigt, daß diese Umsetzung, also die Substitution des 2-exo-Acyl-Restes durch den voluminösen Purin-Rest, infolge sterischer Hinderung unter den erfindungsgemäß angewandten Bedingungen nicht abläuft.

Die in der bisher beschriebenen ersten Stufe des ersten Synthese-Weges entstehenden Verbindungen weisen in 6-Stellung des Purin-Ringsystems im Falle des Adenins eine unsubstituierte Aminogruppe, im Falle des Methylmercaptopurins die $CH_3$-S-Gruppe und im Fall des Chlorpurins den Chlor-Rest auf. Die 6-N-substituierten Adenyl-Derivate werden, ausgehend von den 6-Methylpercapto- bzw. 6-Chlor-purin-Derivaten, die somit wertvolle Zwischenprodukte zur Herstellung der erfindungsgemäßen Verbindungen darstellen, dadurch hergestellt, daß man die Methylmercapto- bzw. Chlor-purin-Derivate mit dem gewünschten primären oder sekundären Alkylamin mit einer geradkettigen oder verzweigten Alkylgruppe mit 1 bis 7 C-Atomen oder einem ω-Phenylalkylamin mit einer Alkylgruppe von 1 bis 7 C-Atomen oder einem entsprechenden ω-Phenylalkylamin, bei dem der Phenylring in p-Stellung halogensubstituierf ist, also beispielsweise mit p-Chlor-benzylamin, p-Chlor-phenyläthylamin, p-Chlorphenylpropylamin, umgesetzt wird. Die in 6-Stellung mit einem heterocyclischen sekundären Amin substituierten Purin-Derivate werden aus den entsprechenden Methylmercapto- bzw. Chlor-purin-Derivaten durch Umsetzung mit dem entsprechenden cyclischen, nicht-aromatischen, gegebenenfalls ein weiteres Heteroatom enthaltenden sekundären Amin, beispielsweise mit Pyrrolidin, Piperidin, Piperazin, Morpholin bzw. deren beliebig substituierten Derivaten, erhalten. In analoger Weise kann das unsubstituierte Adenyl-Derivat durch Umsetzung des 6-Chlor- bzw. 6-Methylmercapto-purinyl-Derivats mit Ammoniak erhalten werden.

Die Umsetzung der Methylmercapto- bzw. Chlor-purin-Derivate mit Ammoniak, dem gewünschten primären oder sekundären Alkylamin, gegebenenfalls p-Chlor-substituierten-ω-Phenyl-alkylamin oder cyclischen, nicht-aromatischen, gegebenenfalls ein weiteres Heteroatom enthaltenden sekundären Amin erfolgt unter erhöhter Temperatur und erhöhtem Druck, vorzugsweise bei einer Temperatur von 100-170 °C und einem Druck von 2-50 at, besonders vorteilhaft unter einem Druck von 2-2o at, unter Inertgas-Atmosphäre ($N_2$ oder Ar), wobei Ammoniak bzw. das entsprechende Amin im Überschuß, vorzugsweise in 2-10 fach molarem Überschuß, gegebenenfalls in Gegenwart eines Lösungsmittels, z. B. Methanol, Äthanol, Butanol, eingesetzt werden.

Anstelle der nachträglichen Substitution der 6-Chlor- bzw. 6-Methylmercapto-Gruppe der Purin-1.4;3.6-dianhydro-hexit-Derivate können die Hexit-acylate in der ersten Stufe auch sofort mit den Alkalisalzen des am 6-N-Atom bereits entsprechend substituierten Adenin-Derivats in analoger Weise umgesetzt werden. Entsprechend substituierte Adenine können, soweit sie nicht literaturbekannt sind, aus 6-Chlor- oder 6-Methylmercapto-purin durch Erhitzen mit den entsprechenden Aminen erhalten werden.

Die im Verlauf des bisher beschriebenen ersten Synthese-Weges entstandenen 5-(7-Theophyllinyl)-, 5-(9-Adenyl)- und 5-(6-N-substituierten-9-adnyl)-isohexid-Derivate weisen in 2- oder 5-Stellung des 1.4;3.6-Dianhydro-hexit-Ringsystems jeweils eine freie Hydroxylgruppe auf. Diese freie Hydroxylgruppe wird in an sich bekannter Weise mit Salpetersäure, mit Nitriersäure oder mit einem Gemisch aus Salpetersäure und Eisessig/Acetanhydrid zu den entsprechenden Mononitraten der allgemeinen Formeln Ia bis Ie verestert.

Anschließend kann man im Fall der 9-Adenyl- oder 6-N-monosubstituierten 9-Adenyl-isohexid-nitrate durch deren Umsetzung mit einem Säurechlorid oder Anhydrid einer aliphatischen, gegebenenfalls methylsubstituierten Monocarbonsäure mit 2 bis 7 C-Atomen, beispielsweise mit Pivaloylchlorid, Essigsäurechlorid, Acetanhydrid oder dergleichen, nach Art einer Schotten-Baumann-Reaktion oder deren Einhorn-Variante die entsprechenden Acyl-Derivate herstellen.

Der bisher beschriebene erste erfindungsgemäße Weg zur Herstellung der erfindungsgemäßen, bei dem der entsprechende Isohexid mit einem Sulfonsäurechlorid, vorzugsweise mit Methansulfonsäurechlorid oder Toluolsulfonsäurechlorid, in den entsprechenden Monoacyl-1.4;3.6-dianhydrohexit übergeführt wird, hat den Nachteil, daß bei der Acylierung nicht nur das entsprechende 5-0-Acyl-Derivat bzw. 2-0-Acyl-Derivat entsteht, sondern gleichzeitig auch das 2,5-Diacyl-Derivat, so daß bei den isoidid- und Isomannid-Derivaten jeweils die Monoacyl-Verbindung vom Diacylat abgetrennt werden muß, während beim Isosorbid, bei dem zwei stereoisomere Monoacyl-Derivate neben dem Diacylat entstehen, das gewünschte Acylat aus dem Gemisch der drei Acyl-Derivate isoliert werden muß. Die Auftrennung des Acylatgemisches erfolgt entweder durch fraktionierte Kristallisation, fraktionierte Extraktion oder mit Hilfe anderer an sich bekannter Methoden.

Die mühsame und aufwendige Auftrennung des Acylat-Gemischs entfällt jedoch bei Anwendung des erfindungsgemäßen zweiten Syntheseweges zu den 5-Purinyl-isoidid-Derivaten dadurch, daß 1.4;3.6-Dianhydro-D-glucit mit einem Überschuß an Sulfonsaürechlorid, vorzugsweise Methansulfonylchlorid oder Toluolsulfonylchlorid, in Pyridin oder Chloroform/Triäthylamin quantitativ zum entsprechenden 1.4;3.6-Dianhydro-D-glucit-2.5-diacylat umgesetzt wird.

Das Diacylat wird dann mit dem Alkali-Salz des Adenins, 6-N-substituierten Adenins oder Theophyllins unter entsprechenden Bedingungen, wie bei dem ersten Syntheseweg beschrieben, umgesetzt, wobei — wegen der oben geschilderten sterischen Hinderung der Substitution in 2-exo-Stellung — nur der 5-endo-Acylat-Rest durch den entsprechenden Purinrest bei gleichzeitiger Inversion der Konfiguration am $C^5$-Atom substituiert wird. Es entsteht also das 5-(9-Adenyl)-, bzw. 5-(6-N-substituierte 9-adenyl)-, bzw. 5-(7-Theophyllinyl)-5-desoxy-1.4;3.6-dianhydro-L-idit-2-acylat, welches durch Hydrolyse zu dem entsprechenden Adenyl-, substituierten Adenyl- bzw. Theophyllinyl-isoidid-Derivat verseift und anschließend — ebenso, wie beim ersten erfindungsgemäßen Syntheseweg — in an sich bekannter Weise mit Salpetersäure, Nitriersäure oder mit einem Gemisch aus Salpetersäure und Eisessig/Acetanhydrid verestert und gegebenenfalls anschließend analog zum ersten Syntheseweg am 6-N-Atom acyliert wird.

Der Grad der sterischen Hinderung bei der nucleophilen Substitution der 2-exo-Acylat-Gruppe im Isosorbid-diacylat ist temperaturabhängig. Um aus dem entsprechenden Diacylat möglichst quantitativ das Purinyl-isoidid -monoazcylat zu erhalten, arbeitet man deshalb erfindungsgemäß vorzugsweise bei einer Temperatur von 80-100 °C, da bei Temperaturen über 100 °C auch die 2-exo-Acylgruppe, wenn auch in geringem Ausmaß, von dem eingesetzten Purin-Derivat angegriffen wird. Als Lösungsmittel werden hierbei vorzugsweise Dimethylsulfoxid oder Dimethylformamid eingesetzt.

Um die erfindungsgemäßen Verbindungen in ihre physiologisch unbedenklichen Salze überzuführen, können anorganische Säuren und Mineralsäuren wie Halogenwasserstoffsäuren und Phosphorsäuren sowie organische Säuren wie Carbon- und Sulfonsäuren, beispielsweise Malon-, Bernstein-, Milchwein-, Äpfel-, Benzoe-, Salicyl-, Citronen-, Ascorbin-, Nicotin- oder p-Toluolsulfonsäure verwendet werden. Die freien Basen können aus den Säureadditionssalzen wieder durch Behandlung mit starken Basen, beispielsweise Natrium- oder Kaliumhydroxid, freigesetzt werden.

Gegenstand der Erfindung sind ferner pharmazeutische Zubereitungen, die neben üblichen Träger- und Zusatzstoffen mindestens eine der erfindungsgemäßen Verbindungen oder ihrer physiologisch unbedenklichen Salze enthalten. Diese Zubereitungen können als Arzneimittel in der Human- und Verterinärmedizin verwendet werden. Übliche Trägerstoffe sind beispielsweise Wasser, pflanzliche Öle, Polyäthylenglykole, Glycerinester, Gelatine, Kohlenhydrate wie Laktose oder Stärke, Magnesiumstearat, Talk, Vaseline. Übliche Zusatzstoffe sind beispielsweise Konservierungs-, Stabilisierungs-, Gleit-, Netzmittel, Emulgatoren, physiologisch unbedenkliche Salze, Puffersubstanzen, Farb-, Geschmacks- und Aromastoffe. Die Auswahl der Träger- und Zusatzstoffe hängt davon ab, ob die erfindungsgemäßen Verbindungen enteral, parenteral oder topikal appliziert werden sollen.

Die erfindungsgemäßen Verbindungen können auch im Gemisch mit anderen Wirkstoffen, beispielsweise Vitaminen oder bekannten, im Handel befindlichen Herz-Kreislauf-Mitteln, insbesondere auch mit β-Rezeptorenblockern, verabreicht werden.

Beispiel für eine pharmazeutische Zubereitung :

Für die Herstellung von Tabletten von je 100 mg Einzelgewicht, die je 5 mg Wirkstoff enthalten, benötigt man

I. 5 g 5-(9-Adenyl)-5-desoxy-1.4;3.6-dianhydro-L-idit-2-nitrat-Hydrochloid-Halbhydrat
II. 54 g mikrokristalline Cellulose
III. 20 g Milchzucker
IV. 20 g Maisstärke
V. 0,5 g kolloidale Kieselsäure
VI. 0,5 g Magnesiumstearat

Die Substanzen I-IV werden trocken 10 Minuten lang gemischt, anschließend wird das Gemisch der Substanzen V und VI zugegeben, man mischt weitere 10 Minuten und verpreßt das so erhaltene Pulver auf einer Tablettiermaschine zu Tabletten von 100 mg Einzelgewicht.

Jede der in den nachfolgenden Beispielen genannten erfindungsgemäßen Verbindungen und Zwischenprodukte stellt ein zur Herstellung pharmazeutischer Zubereitungen besonders geeignetes Mittel dar.

Die in den Beispielen enthaltenen Abkürzungen haben folgende Bedeutungen :

Schmp. = Schmelzpunkt (unkorrigiert)
(Z) = Zerstetzung
d = Dichte
$[\alpha]_D^{25}$ = optische Drehung bei 25 °C, Natrium-D-Linie.

Hinter den optischen Drehwerten sind die Konzentrationen der gemessenen Lösungen angegeben, wobei c 2 beispielsweise eine Konzentration von 2 g/100 ml Lösung bedeutet ; das Lösungsmittel ist jeweils gesondert angegeben.

Alle Temperaturen sind in °C angegeben.

Beispiel Nr. 1

5-(9-Adenyl)-5-desoxy-1.4;3.6-dianhydro-L-idit-2-nitrat :

a) 1.4;3.6-Dianhydro-D-glucit-2-methansulfonat, -5-methansulfonat und -2,5-dimethansulfonat :

Zur Lösung von 4.82 kg (33 Mol) 1.4;3.6-Dianhydro-D-glucit in 24 Liter Pyridin tropft man unter Feuchtigkeitsausschluß, Rühren und Kühlen auf −15° innerhalb mehrerer Stunden 3.1 Liter (40 Mol) Methansulfonsäurechlorid. Anschließend rührt man 15 Stunden ohne Kühlung weiter. Man destilliert im Vak. das Pyridin ab, gibt zum öligen Rückstand 15 Liter Wasser, kocht auf und läßt abkühlen. Absaugen, Waschen mit 4 Litern Wasser und Trocknen des kristallinen Niederschlags ergibt 2.22 kg (7.34 Mol) 1.4;3.6-Dianhydro-D-glucit-2.5-dimethansulfonat. Das Filtrat wird unter Rühren und Wasserkühlung mit ca. 1.5 kg Natriumhydroxid neutralisiert und bei ca. 70° im Vakuum bis zur Trockne eingedampft. Der Trockenrückstand wird mit insgesamt 30 Litern Chloroform kontinuierlich heiß extrahiert und der Extrakt heiß filtriert. Man läßt den Extrakt 15 Stunden bei 20° stehen, saugt den kristallinen Niederschlag ab, wäscht ihn zweimal mit je 2 Litern Chloroform, trocknet und erhält 2.3 kg (10.26 Mol) 1.4;3.6-Dianhydro-D-glucit-5-methansulfonat. Die vereinigten Filtrate werden im Vakuum eingedampft und der Rückstand heiß in 22 Litern Aethanol gelöst. Man läßt 15 Stunden bei 20° stehen, saugt den kristallinen Niederschlag ab, wäscht ihn zweimal mit je 3 Litern Aethanol, trocknet und erhält 0.65 kg (2.90 Mol) 1.4;3.6-Dianhydro-D-glucit-2-methansulfonat. Eindampfen der Filtrate ergibt 2.21 kg (9.85 Mol) eines Gemisches der beiden isomeren Mono-methansulfonate, das je nach Bedarf durch Wiederholung der abwechselnden Kristallisationen aus Chloroform und Aethanol weiter aufgetrennt werden kann, bzw. durch Veresterung mit Methansulfonsäurechlorid in Pyridin vollständig in 1,4;3.6-Dianhydro-D-glucit-2.5-dimethansulfonat übergeführt wird. Analytische Mengen der Methansulfonate ergeben nach Umkristallisation korrekte Elementaranalysen und die in Tabelle 1 aufgeführten Schmelzpunkte und optischen Drehungen :

(Siehe Tabelle 1, Seite 8 f.)

Tabelle 1

| 1.4;3.6-Dianhydro-D-glucit- | Umkristallisiert aus | Schmp. [°C] | $[\alpha]_D^{25}$ |
|---|---|---|---|
| -2-methansulfonat | Chloroform | 135-138.5 | 62.5 (c 2;Aceton) |
| -5-methansulfonat | Chloroform | 123-124 | 75.9 (c 2;Methanol) |
| -2.5-dimethansulfonat | Aethanol/ Aceton | 127-128 | 74 (c 2;Aceton) |

b) 5-(9-Adenyl)-5-desoxy-1.4;3.6-dianhydro-L-idit :

Unter Stickstoffatmosphäre und Rühren tropft man bei 100° zur Suspension von 15.7 g (0.1 Mol) Adenin-Natriumsalz in 200 ml wasserfreiem Dimethylsulfoxid die Lösung von 22.4 g (0.1 Mol) 1.4;3.6-Dianhydro-D-glucit-5-methansulfonat in 100 ml wasserfreiem Dimethylsulfoxid zu und rührt noch 8 Tage bei 100°. Man destilliert in Vakuum das Lösungsmittel ab und extrahiert den Rückstand in der Siedehitze nacheinander mit 100 ml Chloroform und n-Butanol. Der Chloroformextrakt wird auf ca. 300 ml konzentriert, durch Zugabe von 600 ml Petroläther das Rohprodukt ausgefällt und abgetrennt; das Filtrat wird verworfen. Der Butanolextrakt ergibt nach Eindampfen im Vak. weiteres Rohprodukt. Die vereinigten Rohprodukte werden aus 400 ml Aethanol umkristallisiert. Man erhält 9.2 g (35 mmol) 5-(9-Adenyl)-5-desoxy-1.4;3.6-dianhydro-L-idit. Die eingedampfte Mutterlauge wird in 200 ml Wasser gelöst und kontinuierlich mit ca. 1 Liter einer Mischung von Chloroform/Butanol 9/1 extrahiert. Eindampfen des Extraktes und Umkristallisation aus Chloroform ergibt weitere 3.9 g (15 mmol) Reinprodukt.

Schmp. 202.5-204.5°; $[\alpha]_D^{25}$ 22.2 (c 1 ; Wasser)
Elementaranalyse : $C_{11}H_{13}N_5O_3$ (263.25)
Ber. : C (50.19), H (4.98) N (26.60)
Gef. : C (50.39), H (5.04) N (26.69)

c) 5-(9-Adenyl)-5-desoxy-1.4;3.6-dianhydro-L-idit-2-nitrat :

Unter Rühren und Kühlen auf −15° versetzt man 35 ml 95 %ige Salpetersäure (d = 1.5) langsam mit 2.5 g Harnstoff und fügt dann portionenweise insgesamt 4.0 g (15 mmol) 5-(9-Adenyl)-5-desoxy-1.4;3.6-dianhydro-L-idit zu. Man rührt noch 1 Stunde bei −15° und gießt die Mischung unter Kühlen und Rühren langsam in 300 ml kaltes Wasser ein. Anschließend wird durch Zugabe von 85 g Kaliumhydrogencarbonat neutralisiert und fünfmal mit je 200 ml einer Mischung aus Chloroform/Methanol 95/5 extrahiert. Die vereinigten Extrakte werden nach dem Trocken über wfr. Natriumsulfat bei ca. 50° im Vakuum eingedampft und aus Chloroform/Petroläther umkristallisiert. Man erhält 3.8 g (12.3 mmol) 5-(9-Adenyl)-5-desoxy-1.4;3.6-dianhydro-L-idit-2-nitrat von Schmp. 158-160° (Z). Zur Analyse kristallisiert man erneut aus Chloroform/ Petroläther um.

Schmp. 162-3 °Z ; $[\alpha]_D^{25}$ 80.9 (c 2 ; Chloroform)
Elementaranalyse : $C_{11}H_{12}N_6O_5$ (308.26)
Ber. : C (42.86), H (3.93), N (27.26)
Gef. : C (42.38), H (3.93), N (27.38)

Beispiel Nr. 2

5-(9-Adenyl)-5-desoxy-1.4;3.6-dianhydro-L-idit-2-nitrat :

a) 5-(9-Adenyl)-5-desoxy-1.4;3.6-dianhydro-L-idit-2-methansulfonat :

Die Mischung aus 20.15 g (150 mmol) Adenin-Natriumsalz, 30.2 g (100 mmol) 1.4;3.6-Dianhydro-D-glucit-2.5-dimethansulfonat und 300 ml Dimethylsulfoxid wird 24 Stunden unter Rühren auf 100° erhitzt. Nach dem Abkühlen versetzt man mit 600 ml Wasser, saugt den aus Reaktionsprodukt und nicht umgesetztem Dimethansulfonat bestehenden Niederschlag ab und wäscht zweimal mit je 50 ml Wasser nach. Der Niederschlag wird mit 600 ml Chloroform verrührt, wobei das Dimethansulfonat in Lösung geht und reines Produkt zurückbleibt. Weiteres Produkt erhält man durch zweimalige Extraktion der Chloroformphase mit je 100 ml 2-molarer Salzsäure, Neutralisation der Salzsäurephasen mit verd. Natronlauge und Absaugen des dabei entstehenden Niederschlags. Anschließend kristallisiert man aus

Aethanol um und erhält 17.5 g (51.3 mmol) 5-(9-Adenyl)-5-desoxy-1.4;3.6-diahydro-L-idit-2-methansulfonat vom Schmp. 238-240° ; $[\alpha]_D^{25}$ 39.8 (c 1 ; Dimethylformamid).

Elementaranalyse : $C_{12}H_{15}N_5O_5S$ (341.34)
Ber. : C (42.22), H (4.43), N (20.52); S (9.39)
Gef. : C (42.36), H (4.34), N (20.76), S (9.2 )

b) 5-(9-Adenyl)-5-desoxy-1.4;3.6-dianhydro-L-idit :

Zur unter Rückfluß siedenden Mischung von 87 g (255 mmol) 5-(9-Adenyl)-5-desoxy-1.4;3.6-dianhydro-L-idit-2-methansulfonat und 4 Litern Wasser tropft man mit einer Geschwindigkeit von 10 ml/Stunde die Lösung von 30.6 g (765 mmol) Natriumhydroxid in 250 ml Wasser unter Rühren zu und rührt anschließend noch 5 Stunden unter Rückfluß. Nach dem Abkühlen neutralisiert man die Lösung mit 510 mmol Salzsaüre, dampft im Vakuum ein, trocknet den Rückstand azeotrop mit n-Butanol und kocht ihn dreimal mit je 2 Litern n-Butanol aus. Die Butanolextrakte werden im Vakuum eingedampft und aus Aethanol umkristallisiert. Man erhält 41.5 g (158 mmol) 5-(9-Adenyl)-5-desoxy-1.4;3.6-dianhydro-L-idit vom Schmp. 203°. Weitere 10 g (38 mmol) Produkt erhält man durch Eindampfen der Mutterlauge, Extraktion des Rückstandes mit Chloroform und Eindampfen des Chloroformextraktes.

c) 5-(9-Adenyl)-5-desoxy-1.4;3.6-dianhydro-L-idit-2-nitrat :

Unter Rühren und Kühlen auf – 20° gibt man zu 200 ml 95 %iger Salpetersäure (d = 1.5) portionenweise insgesamt 15 g Harnstoff. Bei – 20° setzt man innerhalb 1 Stunde 41 g (156 mmol) 5-(9-Adenyl)-5-desoxy-1.4;3.6-dianhydro-L-idit in kleinen Portionen zu und rührt 1 Stunde bei ca. – 15° nach. Das klare Reaktionsgemisch wird langsam unter Rühren in 600 ml Eiswasser gegossen, wobei die Hauptmenge des Reaktionsproduktes in Form des Hydrogennitrats auskristallisiert. Das Kristallisat wird abgesaugt, zweimal mit je 100 ml Wasser gewaschen, in 500 ml Wasser suspendiert durch Zugabe von 2-molarer Natronlauge in die Base übergeführt, erneut abgesaugt und mit Wasser neutral gewaschen. Weiteres Produkt gewinnt man durch Neutralisation des Filtrats der Eiswasser-Fällung mit Natronlauge (4,4 Mol), Vereinigen mit sämtlichen zuvor erhaltenen Filtraten und Waschwässern, Extration mit zweimal je 1 Liter Chloroform und Eindampfen der Chloroformextrakte im Vakuum. Die vereinigten Rohbasen werden in 500 ml Aethanol gelöst und durch Zugabe von 12 ml 37 %iger Salzsäure (144 mmol) in das Hydrochlorid übergeführt, das auskristallisiert. Man saugt ab, wäscht mit 100 ml Aethanol, konzentriert das Filtrat auf 150 ml und saugt erneut ab und wäscht mit wenig Aethanol nach. Man erhält 43.3 g (126 mmol) 5-(9-Adenyl)-5-desoxy-1.4;3.6-dianhydro-L-idit-2-nitrat als Hydrochlorid, mit 1/2 Mol Kristallwasser.

Schmp. 204-205 °Z ; $[\alpha]_D^{25}$ 28.6 (c 0.5 ; Wasser)
Elementaranalyse : $C_{11}H_{12}N_6O_5 \times HCl \times 1/2\ H_2O$ (353.72)
Ber. : C (37.35), H (3.99), N (23.76), Cl (10.02)
Gef. : C (37.35), H (3.95), N (23.73), Cl (10.1 )

Das Hydrogennitrat hat nach Umkristallisation aus Methanol den Schmp. 188-190° und $[\alpha]_D^{25}$ 26.8 (c 0.5 ; Wasser).

Elementaranalyse : $C_{11}H_{12}N_6O_5 \times HNO_3$ (371.27)
Ber. : C (35.59), H (3.53), N (26.41)
Gef. : C (35.71), H (3.46), N (26.30)

## Beispiel Nr. 3

5-(6-Methylaminopurin-9-yl)-5-desoxy-1.4;3.6-dianhydro-L-idit-2-nitrat :

a) 5-(6-Methylmercaptopurin-9-yl)-5-desoxy-1.4;3.6-dianhydro-L-idit :

Die Lösung von 166 g (1 Mol) 6-Methylmercaptopurin und 40 g (1 Mol) Natriumhydroxid in 1 Liter Methanol wird im Vakuum eingedampft und bei 140 °C bis zur Gewichtskonstanz getrocknet. Man erhält 188 g (1 Mol) des 6-Methylmercaptopurin-9-Natriumsalzes. Diese werden zusammen mit 224 g (1 Mol) 1.4;3.6-Dianhydro-D-glucit-5-methansulfonat in 3 Litern Dimethylformamid suspendiert und 24 Stunden unter Rühren und Stickstoffatmosphäre auf 130° erwärmt. Anschließend destilliert man im Vakuum das Dimethylformamid ab, nimmt den Rückstand in 2 Litern Wasser auf und extrahiert im Rotationsperforator kontinuierlich mit ca. 5 Litern Chloroform. Der Chloroformextrakt wird über wasserfreiem Natriumsulfat geklärt, filtriert und eingedampft. Das verbleibende ölige Reaktionsprodukt erstarrt beim Verreiben mit etwas Wasser zu einem kristallinen Brei, der mit 600 ml Wasser verrührt, abgesaugt und mit 2 mal 75 ml Wasser gewaschen wird. Anschließend trocknet man im Vakuumtrockenschrank bei 120° bis zur Gewischtskonstanz und erhält 147 g (0.5 Mol) 5-(6-Methylmercaptopurin-9-yl)-5-desoxy-1.4;3.6-dianhydro-L-idit. Zur Analyse kristallisiert man aus Chloroform/Toluol um.

Schmp. 146-8° ; $[\alpha]_D^{25}$ 22.5 (c I ; Chloroform)
Elementaranalyse : $C_{12}H_{14}N_4O_3S$ (294.33)
Ber. : C (48.97), H (4.79), N (19.04), S (10.89)
Gef. : C (48.66), H (4.70), N (18.70), S (10.6 )

b) 5-(6-Methylaminopurin-9-yl)-5-desoxy-1.4;3.6-dianhydro-L-idit :

Das Gemisch aus 8.8 g (30 mmol) 5-(6-Methylmercaptopurin-9-yl)-5-desoxy-1.4;3.6-dianhydro-L-idit, 8.5 ml 33 %iger methanolischer Methylamin-Lösung (90 mmol) und 100 ml Wasser wird bei 130° 20 Stunden im geschlossenen Autoklaven gerührt. Nach dem Abkühlen und Entspannen dampft man zur Trockne ein, löst den Rückstand in 50 ml Wasser und extrahiert kontinuierlich mit Chloroform. Eindampfen des über wasserfreiem Natriumsulfat getrockneten Chloroformextrakts ergibt 7.1 g (25.6 mmol) öliges Rohprodukt, das zur Reinigung in das Hydrochlorid übergeführt und aus Methanol umkristallisiert wird.

Schmp. 247-250° (Z) ; $[\alpha]_D^{25}$ 35.9 (c l ; Wasser)

Elementaranalyse : $C_{12}H_{15}N_5O_3 \times HCl$ (313.74)

Ber. : C (45.94), H (5.14), N (22.32), Cl (11.30)

Gef. : C (46.08), H (5.25), N (22.29), Cl (11.3 )

c) 5-(6-Methylaminopurin-9-yl)-5-desoxy-1.4;3.6-dianhydro-L-idit-2-nitrat :

Zu der Mischung aus 20 ml Eisessig, 2,5 g (9 mmol) 5-(6-Methyl-aminopurin-9-yl)-5-desoxy-1.4;3.6-dianhydro-L-idit und 0.6 g (10 mmol) Harnstoff tropft man unter Rühren und Kühlen auf 10-15° die Lösung von 1.77 ml (40 mmol) 95 %iger Salpetersäure (d = 1.5) in 10 ml Eisessig und anschließend 10 ml Acetanhydrid zu. Man rührt 3 Stunden bei 10-15° nach, verdünnt mit Wasser auf 200 ml Gesamtvolumen, rührt unter Zugabe von 5 g Natriumhydrogencarbonat ca. 30 Min., saugt das als feinkörniger Niederschlag ausfallende Reaktionsprodukt ab, wäscht mit 20 ml Wasser und trocknet. Man erhält 1.4 g (3.6 mmol) 5-(6-Methylaminopurin-9-yl)-5-desoxy-1.4;3.6-dianhydro-L-idit-2-nitrat in Form des Hydrogennitrats. Weitere 0.7 g (1.8 mmol) Produkt gewinnt man durch Extraktion des Filtrats mit 3 mal 100 ml Chloroform, Waschen des Chloroformextrakts mit 50 ml Wasser, Eindampfen im Vakuum und Umfällen des Rückstandes aus Aether/Petroläther. Die analytische Probe wird aus Wasser/Isopropanol umkristallisiert.

Schmp. 193-197°(Z) ; $[\alpha]_D^{25}$ 28.6 (c 1.1 ; Wasser).

Elementaranalyse : $C_{12}H_{14}N_6O_5 \times HNO_3$ (385.30)

Ber. : C (37.40), H (3.92), N (25.45)

Gef. : C (37.34), H (3.94), N (25.21)

Ein Teil des Produkts wird in das Hydrochlorid übergeführt und aus Aethanol/Isopropanol umkristallisiert.

Schmp. 201-204° (Z) ; $[\alpha]_D^{25}$ 29.1 (c 1 ; Wasser)

Elementaranalyse : $C_{12}H_{14}N_6O_5 \times HCl$ (358.74)

Ber. : C (40.18), H (4.21), N (23.43), Cl (9.88)

Gef. : C (40.35), H (4.24), N (23.49), Cl (9.9 )

## Beispiel Nr. 4

5-(6-Dimethylaminopurin-9-yl)-5-desoxy-1.4;3.6-dianhydro-L-idit-2-nitrat :

a) 5-(6-Dimethylaminopurin-9-yl)-5-desoxy-1.4;3.6-dianhydro-L-idit :

Die Mischung aus 8.8 g (30 mmol) 5-(6-Methylmercaptopurin-9-yl)-5-desoxy-1.4;3.6-dianhydro-L-idit und 50 ml 40 %iger wäßriger Dimethylamin-Lösung wird im geschlossenen Autoklaven 20 Stunden bei 130° gerührt. Nach dem Abkühlen und Entspannen dampft man im Vakuum ein und kristallisiert aus 20 ml Aethanol um. Man erhält 5.68 g (19.5 mmol) 5-(6-Dimethylaminopurin-9-yl)-5-desoxy-1.4;3.6-dianhydro-L-idit. Die analystische Probe wird aus Toluol umkristallisiert.

Schmp. 139-141° ; $[\alpha]_D^{25}$ 29,5 (c 1 ; Chloroform)

Elementaranalyse : $C_{13}H_{17}N_5O_3$ (291.31)

Ber. : C (53.60), H (5.88), N (24.04)

Gef. : C (53.90), H (5.97), N (24.11)

b) Die Veresterung mit Salpetersäure in Eisessig/Acetanhydrid erfolgt analog zum vorhergehenden Beispiel 3c. Man erhält aus 4.4 g (15.1 mmol) 5-(6-Dimethylaminopurin-9-yl)-5-desoxy-1.4;3.6-dianhydro-L-idit insgesamt 3.35 g (10 mmol) 5-(6-Dimethylaminopurin-9-yl)-5-desoxy-1.4;3.6-dianhydro-L-idit-2-nitrat. Die analytische Probe wird aus Toluol/Petroläther umkristallisiert.

Schmp. 134-136° (Z) ; $[\alpha]_D^{25}$ 54.2 (C 0.5 ; Chloroform).

Elementaranalyse : $C_{13}H_{16}N_6O_5$ (336.31)

Ber. : C (46.43), H (4.80), N (24.99)

Gef. : C (46.38), H (4.89), N (24.69)

Ein Teil wird in das Hydrochlorid übergeführt und aus Isopropanol/Aethanol umkristallisiert.

Schmp. 169-170° (Z) ; $[\alpha]_D^{25}$ 37.5 (c 1.1 ; Wasser) $C_{13}H_{16}N_6O_5$ x HCl (372.77).

Beispiel Nr. 5

5-(6-Aethylaminopurin-9-yl)-5-desoxy-1.4;3.6-dianhydro-L-idit-2-nitrat :

a) 5-(6-Aethylaminopurin-9-yl)-5-desoxy-1.4;3.6-dianhydro-L-idit :

Die Mischung aus 8.8 g (30 mmol) 5-(6-Methylmercaptopurin-9-yl)-5-desoxy-1.4;3.6-dianhydro-L-idit, 8.1 g (100 mmol) Aethylamin-Hydrochlorid, 4.0 g (100 mmol) Natriumhydroxid und 50 ml Wasser wird im geschlossenen Autoklaven 20 Stunden bei 130° gerührt. Nach dem Abkühlen und Entspannen dampft man zur Trockne ein, löst den Rückstand in 100 ml Wasser und extrahiert 10 mal mit je 100 ml Chloroform. Eindampfen der Chloroformextrakte ergibt 8.15 g öliges Rohprodukt, das mit 14 ml 2-molarer Salzsäure in das Hydrochlorid übergeführt, erneut eingedampft und aus Methanol/Aether umkristallisiert wird. Man erhält 5,7 g (17.4 mmol) 5-(6-Aethylaminopurin-9-yl)-5-desoxy-1.4;3.6-dianhydro-L-idit-Hydrochlorid.

Schmp. 240°-2° ; $[\alpha]_D^{25}$ 37.7 (c 1 ; Wasser)

Elementaranalyse : $C_{13}H_{17}N_5O_3$ x HCl (327.76)

Ber. : C (47.64), H (5.53), N (21.37), Cl (10.82)

Gef. : C (47.78), H (5.61), N (21.40), Cl (10.95)

b) Zur Veresterung mit Salpetersäure überführt man das zuvor erhaltene Hydrochlorid in die Base und behandelt dann analog Beispiel 3 c mit Salpetersäure/Eisessig/Acetanhydrid. Aus 4,4 g Base (15.1 mmol) erhält man 2.8 g (7 mmol) 5-(6-Aethylaminopurin-9-yl)-5-desoxy-1.4;3.6-dianhydro-L-idit-2-nitrat in Form des Hydrogennitrats. Die analytische Probe wird aus Methanol umkristallisiert.

Schmp. 185-7° (Z) ; $[\alpha]_D^{25}$ 31.7 (c 0.5 ; Wasser)

Elementaranalyse : $C_{13}H_{16}N_6O_5$ x $HNO_3$ (399.33)

Ber. : C (39.10), H (4.29), N (24.55)

Gef. : C (39.14), H (4.37), N (24.30)

Ein Teil wird in das Hydrochlorid übergeführt und aus Isopropanol/Aethanol umkristallisiert.

Schmp. 212-14° (Z) ; $[\alpha]_D^{25}$ 48.5 (c 1 ; Wasser)

Elementaranalyse : $C_{13}H_{16}N_6O_5$ x HCl (372.77)

Ber. : C (41.89), H (4.60), N (22.54), Cl (9.51)

Gef. : C (42.08), H (4.60), N (22.61), Cl (9.4 )

Beispiel Nr. 6

5-(6-Pyrrolidinopurin-9-yl)-5-desoxy-1.4;3.6-dianhydro-L-idit-2-nitrat :

a) 5-(6-Pyrrolidinopurin-9-yl)-5-desoxy-1.4;3.6-dianhydro-L-idit :

Das Gemisch aus 8.8 g (30 mmol) 5-(6-Methylmercaptopurin-9-yl)-5-desoxy-1.4;3.6-dianhydro-L-idit und 25 ml (300 mmol) Pyrrolidin wird 20 Stunden im geschlossenen Autoklaven bei 130° gerührt. Nach dem Abkühlen und Entspannen destilliert man überschüssiges Pyrrolidin ab, gegen Ende unter Zugabe von Wasser, und kristallisiert den Rückstand aus Wasser um. Man erhält 8.43 g (26.6 mmol) 5-(6-Pyrrolidinopurin-9-yl)-5-desoxy-1.4;3.6-dianhydro-L-idit.

Schmp. 183.5-185.5° ; $[\alpha]_D^{25}$ 34.5 (c 1 ; Chloroform)

Elementaranalyse : $C_{15}H_{19}N_5O_3$ (317.35)

Ber. : C (56.77), H (6.03), N (22.07)

Gef. : C (57.00), H (6.07), N (22.08)

b) Veresterung mit Salpetersäure :

Zur Lösung von 6.35 g (20 mmol) des zuvor erhaltenen 5-(Pyrrolidinopurin-9-yl)-5-desoxy-1.4;3.6-dianhydro-L-idit in 40 ml Essigsäure gibt man 1.2 g (20 mmol) Harnstoff und tropft unter Rühren und Kühlen auf ca. 10-15° die Lösung von 3.5 ml (80 mmol) 95 %iger Salpetersäure (d = 1.5) in 20 ml Eisessig und anschließend noch 20 ml Acetanhydrid zu. Man rührt noch 5 Stunden bei 15-20° nach, verdünnt dann mit Wasser auf 270 ml Gesamtvolumen, rührt 30 Min. und gibt portionenweise 6 g (71 mmol) Natriumhydrogencarbonat zu. Man extrahiert dreimal mit je 100 ml Chloroform, trocknet die Chloroformextrakte über wasserfreiem Natriumsulfat und Natriumcarbonat und dampft im Vakuum bei ca. 40-50° ein. Der Rückstand wird in 100 ml Aether gelöst ; durch Zugabe von Petroläther fällt man Nebenprodukte als öligen Niederschlag aus. Man dekantiert vom Niederschlag ab und gibt weiteren Petroläther zu, wobei das Reinprodukt langsam auskristallisiert. Man erhält 5.53 g (15.3 mmol) 5-(6-Pyrrolidinopurin-9-yl)-5-desoxy-1.4;3.6-dianhydro-L-idit-2-nitrat. Die analytische Probe wird aus Toluol/Petroläther umkristallisiert.

Schmp. 146-7° ; $[\alpha]_D^{25}$ 58.4 (c 0.48 ; Chloroform).

Elementaranalyse : $C_{15}H_{18}N_6O_5$ (362.35)

Ber. : C (49.72), H (5.01), N (23.19)

Gef. : C (49.73), H (5.08), N (23.04)

Ein Teil wird in das Hydrochlorid übergeführt und aus Isopropanol/Aether umgefällt.
Schmp. 189-196° (Z) ; $[\alpha]_D^{25}$ 44.7 (c 0.53 ; Wasser)
Elementaranalyse : $C_{15}H_{18}N_6O_5$ x HCl (398.81)
Ber. : C (45.18), H (4.80), N (21.07), Cl (8.89)
Gef. : C (45.20), H (4.83), N (21.17), Cl (9.1 )

Beispiel Nr. 7

5-(6-Piperidinopurin-9-yl)-5-desoxy-1.4;3.6-dianhydro-L-idit-2-nitrat

a) 5-(6-Piperidinopurin-9-yl)-5-desoxy-1.4;3.6-dianhydro-L-idit :
Analog zum vorgehenden Beispiel werden 8.8 g (30 mmol) 5-(6-Methylmercaptopurin-9-yl)-5-desoxy-1.4;3.6-dianhydro-L-idit zusammen mit 25 ml (253 mmol) Piperidin 20 Stunden bei 130° im geschlossenen Autoklaven gerührt. Nach Abdestillieren des überschüssigen Piperidins (gegen Ende unter Zugabe von Wasser) verreibt man den öligen Rückstand mit Toluol. Das nun feste Rohprodukt wird abgesaugt, getrocknet und aus Wasser umkristallisiert. Man erhält 7,7 g (23.2 mmol) reinen 5-(6-Piperidinopurin-9-yl)-5-desoxy-1.4;3.6-dianhydro-L-idit.
Schmp. 136-139° ; $[\alpha]_D^{25}$ 30.1 (c 1 ; Chloroform)
Elementaranalyse : $C_{16}H_{21}N_5O_3$ (331.38)
Ber. : C (57.99), H (6.39), N (21.13)
Gef. : C (58.14), H (6.48), N (21.13)

b) Die Veresterung des vorstehend beschriebenen Produkts mit Salpetersäure in Eisessig/Acetanhydrid erfolgt analog Beispiel 6 b. Aus 5.0 g (15 mmol) erhält man nach Kristallisation aus Aether/Petroläther 2.17 g (5.8 mmol) 5-(6-Piperidinopurin-9-yl)-5-desoxy-1.4;3.6-dianhydro-L-idit-2-nitrat. Die analytische Probe wird aus Toluol/Petroläther umkristallisiert.
Schmp. 115-117° ; $[\alpha]_D^{25}$ 58.1 (c 1 ; Chloroform)
Elementaranalyse : $C_{16}H_{20}N_6O_5$ (376.38)
Ber. : C (51.06), H (5.36), N (22.33)
Gef. : C (50.98), H (5.47), N (22.20)

Ein Teil des Produkts wird in das Hydrochlorid übergeführt und aus Isopropanol/Aether umgefällt.
Schmp. 179-184° (Z) ; $[\alpha]_D^{25}$ 58 (c 0.5 ; Methanol)
Elementaranalyse : $C_{16}H_{20}N_6O_5$ x HCl (412.84)
Ber. : C (46.55), H (5.13), N (20.36), Cl (8.59)
Gef. : C (46.68), H (5.22), N (20.43), Cl (8.6 )

Beispiel Nr. 8

5-(6-Morpholinopurin-9-yl)-5-desoxy-1.4;3.6-dianhydro-L-idit-2-nitrat :

a) 5-(6-Morpholinopurin-9-yl)-5-desoxy-1.4;3.6-dianhydro-L-idit :
Analog zu den voranstehenden Beispielen 6 a und 7 a erhält man aus 8.8 g (30 mmol) 5-(6-Methylmercaptopurin-9-yl)-5-desoxy-1.4;3.6-dianhydro-L-idit und 25 ml (287 mmol) Morpholin nach 20 Stunden Rühren bei 130° im geschlossenen Autoklaven, Abdestillieren des überschüssigen Morpholins unter Wasserzugabe und Umkristallisation des Rückstandes aus Wasser 6.9 g (18.7 mmol) reinen 5-(6-Morpholinopurin-9-yl)-5-desoxy-1.4;3.6-dianhydro-L-idit in Form des Dihydrats.
Schmp. 84-88° ; $[\alpha]_D^{25}$ 26.4 (c 1 ; Chloroform)
Elementaranalyse : $C_{15}H_{19}N_5O_4$ X 2 $H_2O$ (369.38)
Ber. : C (48.78), H (6.28), N (18.96)
Gef. : C (48.71), H (6.31), N (18.74)

b) Veresterung mit Salpetersäure :
Analog zu den Beispielen 6 b und 7 b erhält man aus 5.5 g (14.5 mmol) des voranstehend beschriebenen Dihydrats durch Behandeln mit 95 %iger Salpetersäure in Eisessig/Acetanhydrid und Umkristallisation des Rohprodukts aus wenig Aethanol 3.65 g (9.6 mmol) 5-(6-Morpholinopurin-9-yl)-5-desoxy-1.4;3.6-dianhydro-L-idit-2-nitrat. Die analytische Probe wird nochmal aus Toluol/Aether umkristallisiert.
Schmp. 173-5° ; $[\alpha]_D^{25}$ 53.8 (c 1 ; Chloroform)
Elementaranalyse : $C_{15}H_{18}N_6O_6$ (378.35)
Ber. : C (47.62), H (4.79), N (22.21)
Gef. : C (48.16), H (4.92), N (21.72)

Ein Teil des Produkt wird in das Hydrochlorid übergeführt und aus Isopropanol/Aether umgefällt.

Schmp. 187-9° (Z) ; $[\alpha]_D^{25}$ 55.0 (c 0.51 ; Methanol)
Elementaranalyse : $C_{15}H_{18}N_6O_6$ x HCl (414.81)
Ber. : C (43.43), H (4.62), N (20.26), Cl (8.55)
Gef. : C (44.20), H (4.72), N (20.25), Cl (8.1 )

Beispiel Nr. 9

5-[6-(4-Methylpiperazino)-purin-9-yl]-5-desoxy-1.4;3.6-dianhydro-L-idit-2-nitrat :

a) 5-[6-(4-Methylpiperazino)-purin-9-yl)-5-desoxy-1.4;3.6-dianhydro-L-idit :
Analog zu den vorgehenden Beispielen erhält man aus 8.8 g (30 mmol) 5-(6-Methylmercaptopurin-9-yl)-5-desoxy-1.4;3.6-dianhydro-L-idit und 25 ml (225 mmol) N-Methylpiperazin 10.2 g (29.4 mmol) Rohprodukt. Umkristallisation aus Toluol ergibt 6.3 g (18.2 mmol) Reinprodukt.
Schmp. 166-7° ; $[\alpha]_D^{25}$ 31.1 (c 1 ; Chloroform)
Elementaranalyse : $C_{16}H_{22}N_6O_3$ (346.40)
Ber. : C (55.48), H (6.40), N (24.26)
Gef. : C (55.60), H (6.45), N (23.91)

b) Die Veresterung mit Salpetersäure wird analog zu den vorangehenden Beispielen durchgeführt. Nach Umkristallisation aus Aether erhält man 5-[6-(4-Methylpiperazino)-purin-9-yl]-5-desoxy-1,4;3.6-dianhydro-L-idit-2-nitrat.
Schmp. 107-9° ; $[\alpha]_D^{25}$ 62.1 (c 0.53 ; Chloroform)
Elementaranalyse : $C_{16}H_{21}N_7O_5$ (391.40)
Ber. : C (49.10), H (5.41), N (25.05)
Gef. : C (49.40), H (5.59), N (24.93)

Ein Teil des Produkts wird mit 2 Aequivalenten Salzsäure versetzt, im Vakuum eingedampft und aus Aethanol/Isopropanol umkristallisiert. Man erhält das Dihydrochlorid-Hydrat.
Schmp. 173-80° (Z) ; $[\alpha]_D^{25}$ 32 (c 1 ; Wasser)
Elementaranalyse : $C_{16}H_{21}N_7O_5$ x 2HCl x $H_2O$ (482.34)
Ber. : C (39.84), H (5.22), N (20.33), Cl (14.70)
Gef. : C (40.39), H (5.39), N (20.50), Cl (14.6 )

Beispiel Nr. 10

5-[9-(6-N-Pivaloyl)-adenyl]-5-desoxy-1.4;3.6-dianhydro-L-idit-2-nitrat

Die Lösung von 3.1 g (10 mmol) 5-(9-Adenyl-5-desoxy-1.4;3.6-dianhydro-L-idit-2-nitrat und 7 ml (50 mmol) Triäthylamin in 80 ml wasserfreiem Dioxan wird unter Rühren und Feuchtigkeitsausschluß mit 6.1 ml (50 mmol) Pivaloylchlorid versetzt. Man erhitzt 1 Stunde unter Rückfluß, läßt abkühlen und saugt ausgefallenes Triäthylamin-Hydrochlorid ab. Das Filtrat wird auf ca. 30 ml Volumen eingedampft und in 100 ml Eiswasser eingerührt. Der sich abscheidende ölige Niederschlag wird abgetrennt und die wäßrige Phase mit 50 ml Chloroform extrahiert. Niederschlag und Chloroformextrakt werden vereinigt ; aus der so erhaltenen Lösung fällt man das Rohprodukt mit Petroläther aus. Umfällen des Rohprodukts aus Chloroform/Petroläther, gefolgt von der Umkristallisation aus Aethanol ergibt 1.83 g (4.7 mmol) 5-[9-(6-N-Pivaloyl)-adenyl]-1.4;3.6-dianhydro-L-idit-2-nitrat.
Schmp. 171-3° ; $[\alpha]_D^{25}$ 35.8 (c 0.95 ; Chloroform)
Elementaranalyse : $C_{16}H_{20}N_6O_6$ (392.38)
Ber. : C (48.98), H (5.14), N (21.42)
Gef. : C (48.96), H (5.24), N (20.61)

Beispiel Nr. 11

5-[9-(6-N-Acetyl)-adenyl]-5-desoxy-1.4;3.6-dianhydro-L-idit-2-nitrat :

Das Produkt wird analog zu Beispiel Nr. 10 durch Acetylierung von 5-(9-Adenyl)-5-desoxy-1.4;3.6-dianhydro-L-idit-2-nitrat erhalten. Nach Umkristallisation aus Methanol/Aether hat es den Schmp. 158-161° und $[\alpha]_D^{25}$ 35 (c 1 ; Chloroform). $C_{13}H_{14}N_6O_6$ (350.29)

Beispiel Nr. 12

5-(7-Theophyllinyl)-5-desoxy-1.4;3.6-dianhydro-L-idit-2-nitrat :

a) 5-(7-Theophyllinyl)-5-desoxy-1.4;3.6-dianhydro-L-idit :
Die Mischung aus 44.8 g (0.2 Mol) 1.4;3.6-Dianhydro-D-glucit-5-methansulfonat, 80.8 g (0.4 Mol) Theophyllin-Natriumsalz und 600 ml Dimethylsulfoxid wird 48 Stunden unter Stickstoffatmosphäre bei 130° gerührt. Man destilliert das Dimethylsulfoxid im Vakuum ab, löst den Rückstand in 400 ml heißem Wasser, extrahiert nach dem Abkühlen zweimal mit je 200 ml Chloroform und schüttelt die Chloroformextrakte mit 200 ml Wasser aus. Die vereinigten Wasserphasen werden mit 30 g Aktivkohle aufgekocht, filtriert und das Filtrat nach Neutralisation zur Trockne eingedampft. Der Rückstand wird mit 1 Liter Aethanol gerührt, filtriert und erneut eingedampft. Zur Entfernung restlichen Theophyllins löst man dann in 700 ml 2 %iger Natronlauge und extrahiert das Reaktionsprodukt kontinuierlich mit Chloroform. Der Chloroformextrakt ergibt nach dem Trocknen über wasserfreiem Natriumsulfat und Eindampfen 27.5 g (89 mmol) 5-(7-Theophyllinyl)-5-desoxy-1.4;3.6-dianhydro-L-idit. Die analytische Probe wird aus Aceton/Aethanol umkristallisiert.

Schmp. 196-8° ; $[\alpha]_D^{25}$ – 27.9 (c 0,5 ; Wasser)
Elementaranalyse : $C_{13}H_{16}N_4O_5$ (308.30)
Ber. : C (50.65), H (5.23), N (18.17)
Gef. : C (50.68), H (5.32), N (18.06)

b) Veresterung mit Salpetersäure :

Zu 150 ml 95 %iger Salpetersäure (d = 1.5) gibt man unter Rühren und Kühlen auf – 15° in kleinen Portionen zunächst 10.8 g (180 mmol) Harnstoff und anschließend ebenfalls in kleinen Portionen insgesamt 17.45 g (56.6 mmol) des voranstehend beschriebenen 5-(7-Theophyllinyl)-5-desoxy-1.4;3.6-dianhydro-L-idit. Man rührt 1 Stunde bei – 15° nach, verdünnt mit 850 ml Wasser, gibt eine Lösung von 130 g (3.25 Mol) Natriumhydroxid in 1 Liter Wasser zu und stellt durch Zugabe von Natriumhydrogencarbonat neutral. Das Produkt fällt als Niederschlag aus ; man saugt ab, wäscht mit 100 ml Wasser und löst den Niederschlag in 1 Liter Chloroform.

Nach dem Trocknen über wasserfreiem Natriumsulfat, Filtrieren und Eindampfen der Chloroformlösung kristallisiert man aus Aceton/Methanol um und erhält 14.5 g (41 mmol) 5-(7-Theophyllinyl)-5-desoxy-1.4;3.6-dianhydro-L-idit-2-nitrat.

Schmp. 193° ; $[\alpha]_D^{25}$ 22.9 (c 0.5 ; Dimethylformamid)
Elementaranalyse : $C_{13}H_{15}N_5O_7$ (353.29)
Ber. : C (44.20), H (4.28), N (19.82)
Gef. : C (44.07), H (4.27), N (19.58)

## Beispiel Nr. 13

5-(6-Benzylaminopurin-9-yl)-5-desoxy-1.4;3.6-dianhydro-L-idit-2-nitrat :

a) 5-(6-Benzylaminopurin-9-yl)-5-desoxy-1.4;3.6-dianhydro-L-idit :

Das Gemisch aus 8.8 g (30 mmol) 5-(6-Methylmercaptopurin-9-yl)-5-desoxy-1.4;3.6-dianhydro-L-idit und 25 ml (229 mmol) Benzylamin wird im geschlossenen Autoklaven 20 Stunden bei 130° gerührt. Nach dem Abkühlen und Entspannen destilliert man das überschüssige Benzylamin — gegen Ende unter Zugabe von Wasser — im Vakuum ab, kristallisiert den öligen Rückstand aus Aceton um und erhält, 6.9 g (19.5 mmol) 5-(6-Benzylaminopurin-9-yl)-5-desoxy-1.4;3.6-dianhydro-L-idit. Die analytische Probe wird nochmal aus Toluol umkristallisiert.

Schmp. 151-3° ; $[\alpha]_D^{25}$ 28.7 (c 1 ; Chloroform)
Elementaranalyse : $C_{18}H_{19}N_5O_3$ (353.38)
Ber. : C (61.18), H (5.42), N (19.82)
Gef. : C (61.17), H (5.44), N (19.82)

b) Veresterung mit Salpetersäure :

Zu der Mischung aus 5.3 g (15 mmol) des zuvor beschriebenen 5-(6-Benzylaminpurin-9-yl)-5-desoxy-1.4;3.6-dianhydro-L-idit, 0.9 g (15 mmol) Harnstoff und 60 ml Essigsäure tropft man unter Rühren und Kühlen auf 10° die Lösung von 2.5 ml (60 mmol) 95 %iger Salpetersäure (d = 1.5) in 15 ml Essigsäure und danach 15 ml Acetanhydrid zu. Man rührt noch 3 Stunden bei 10°, gibt 350 ml Wasser zu, rührt 30 Min. und gibt dann 5 g Natriumhydrogencarbonat zu. Das Rohprodukt fällt als Niederschlag aus, der abgesaugt, getrocknet und aus Benzol/Aether/Petroläther umkristallisiert wird. Man erhält 4.05 g (ca. 10 mmol) Kristallisat, das aus einem Gemisch von freier Base und Hydrogennitrat des 5-(6-Benzylaminopurin-9-yl)-5-desoxy-1.4;3.6-dianhydro-L-idit-2-nitrat besteht. Ein Teil davon wird in Eisessig gelöst, mit der knapp äquimolaren Menge Salpetersäure versetzt, das auskristallisierende Hydrogennitrat abgesaugt und aus Methanol umkristallisiert.

Schmp. 109-110° ; $[\alpha]_D^{25}$ 57.9 (c 0.54 ; Chloroform)
Elementaranalyse : $C_{18}H_{18}N_6O_5$ x $HNO_3$ (461.40)
Ber. : C (46.86), H (4.15), N (21.25)
Gef. : C (46.56), H (4.20), N (21.08)

Ein weiterer Teil wird zunächst in die freie Base, dann mit Salzsäure in das Hydrochlorid übergeführt und aus Aethanol/Methanol umkristallisiert.

Schmp. 193-201° (Z) ; $[\alpha]_D^{25}$ 52.7 (c 1 ; Methanol)
Elementaranalyse : $C_{18}H_{18}N_6O_5$ x HCl (434.84)
Ber. : C (49.72), H (4.40), N (19.33), Cl (8.15)
Gef. : C (49.82), H (4.40), N (19.36), Cl (8.2 )

## Beispiel Nr. 14

5-[6-(3-Phenylpropyl)-aminopurin-9-yl]-5-desoxy-1.4;3.6-dianhydro-L-idit-2-nitrat :

a) 5-[6-(3-Phenylpropyl)-aminopurin-9-yl]-5-desoxy-1.4;3.6-dianhydro-L-idit :

Das Gemisch aus 19.1 g (65 mmol) 5-(6-Methylmercaptopurin-9-yl)-5-desoxy-1.4;3.6-dianhydro-L-idit und 94 ml (650 mmol) 3-Phenylpropylamin wird im geschlossenen Stahlautoklaven 36 Stdn. auf 150° erhitzt. Nach dem Abkühlen und Entspannen gibt man 80 ml Essigsäure und 500 ml Wasser zu und extrahiert zweimal mit je 300 ml Chloroform. Die Chloroformextrakte werden mit 300 ml Wasser gewaschen und zweimal mit der Lösung von 25 ml 37 %iger Salzsäure in 200 ml Wasser extrahiert. Die salzsauren Extrakte werden mit Natronlauge alkalisiert und mit 400 ml Dichlormethan extrahiert. Der Dichlormethanextrakt ergibt nach dem Trocknen über wasserfreiem Natriumsulfat, Filtrieren und Eindampfen 20 g (52.4 mmol) Rohbase, die in 150 ml Isopropanol in der Wärme gelöst und mit 15 ml 37 %iger Salzsäure (180 mmol) versetzt werden. Beim Abkühlen kristallisieren 18.8 g (41.4 mmol) reines Produkt in Form des Dihydrochlorids aus.

Schmp. 159-62° ; $[\alpha]_D^{25}$ 30.6 (c 0.4 ; Wasser)
Elementaranalyse : $C_{20}H_{23}N_5O_3$ x HCl (454.36)
Ber. : C (52.86), H (5.55), N (15.41), Cl (15.61)
Gef. : C (53.23), H (5.55), N (15.41), Cl (14.4 )

b) Veresterung mit Salpetersäure :

Zur Mischung aus 11.4 g (30 mmol) 5-[6-(3-Phenylpropyl)-aminopurin-9-yl]-5-desoxy-1.4;3.6-dianhydro-L-idit (gewonnen aus dem zuvor beschriebenen Dihydrochlorid), 1.8 g (30 mmol) Harnstoff und 120 ml Eisessig tropft man unter Rühren und Kühlen auf 10-15° die Lösung von 5.2 ml (120 mmol) 96 %iger Salpetersäure (d = 1.5) in 30 ml Eisessig und anschließend 30 ml Acetanhydrid zu, rührt 8 Stdn. bei 15-20° nach, verdünnt mit 800 ml Wasser und läßt über Nacht stehen. Nach Zugabe von 75 ml 2-molarer Natronlauge rührt man gut durch, extrahiert 2 mal mit je 500 ml Chloroform, wäscht die vereinigten Chloroformextrakte mit verdünnter Natronlauge säurefrei, trocknet über wasserfr. Natriumsulfat, filtriert und dampft unter reduziertem Druck ein. Man erhält 11.8 g (ca. 25 mmol) Rohprodukt, das durch am Phenylrest nitriertes Produkt verunreinigt ist. Zur Reinigung überführt man in äthanolischer Lösung mit 27 mmol Salzsäure in das Hydrochlorid, dampft erneut ein, extrahiert die Nebenprodukte in der Siedehitze mit Chloroform und kristallisiert das unlösliche Hauptprodukt aus Aethanol/Chloroform und aus Aethanol um. Das so erhaltene 5-[6-(3-Phenylpropyl)-aminopurin-9-yl]-5-desoxy-1.4;3.6-dianhydro-L-idit-2-nitrat-Hydrochlorid hat den Schmp. 200-203° (Z) ; $[\alpha]_D^{25}$ 41.2 (c 0.21 ; Dimethylformamid).

Ausbeute : 4.84 g (10.5 mmol)
Elementaranalyse : $C_{20}H_{22}N_6O_5$ x HCl (462.89)
Ber. : C (51.90), H (5.01), N (18.16), Cl (7.66)
Gef. : C (52.00), H (5.07), N (18.26), Cl (8.0 )

## Beispiel Nr. 15

2-(9-Adenyl)-2-desoxy-1.4;3.6-dianhydro-D-glucit-5-nitrat :

a) 1.4;3.6-Dianhydro-D-mannit-2-methansulfonat :

Der Lösung von 877 g (6 Mol) 1.4;3.6-Dianhydro-D-mannit in 6 Litern Pyridin tropft man unter Rühren und Feuchtigkeitsausschluß sowie Kühlen auf − 15° innerhalb 6 Stdn. 525 ml (6.6 Mol) Methansulfonylchlorid zu, rührt weitere 3 Tage bei − 15° und destilliert dann unter vermindertem Druck das Pyridin ab. Beim Versetzen des öligen Rückstands mit 2.7 Litern Wasser kristallisiert reines 1.4;3.6-Dianhydro-D-mannit-2.5-dimethansulfonat aus, das abgetrennt und 2 mal mit je 700 ml Wasser gewaschen wird. Die vereinigten Filtrate werden mit einer Lösung von 264 g (6.6 Mol) Natriumhydroxid in 2.5 Litern Wasser versetzt, durch Zugabe von Natriumhydrogencarbonat auf pH = 7 eingestellt, unter reduziertem Druck eingedampft und aceotrop mit Chloroform getrocknet. Der Rückstand wird zweimal mit je 2.5 Litern Chloroform heiß extrahiert und filtriert. Die vereinigten Chloroformextrakte werden 5 mal mit je 1 Liter Wasser extrahiert. Beim Konzentrieren der Wasserphasen kristallisiert das 1.4;3.6-Dianhydro-D-mannit-2-methansulfonat aus. Die nach dem Absaugen verbleibende Mutterlauge ergibt nach dem Eindampfen und Umkristallisieren aus Aethanol weiteres Produkt. Restliches Produkt wird durch Eindampfen der aethanolischen Mutterlauge, Lösen der Rückstandes in Wasser und kontinuierliche Extraktion der wäßrigen Lösung mit Chloroform im Rotationsperforator gewonnen. In der Wasserphase verbleibt nicht umgesetzter 1.4;3.6-Dianhydro-D-mannit. Insgesamt erhält man 396 g (1.77 Mol) 1.4;3.6-Dianhydro-D-mannit-2-methansulfonat (neben 465 g = 1.54 Mol des Dimethansulfonats). Die analytische Probe hat nach Umkristallisation aus Chloroform den Schmp. 111-112° und $[\alpha]_D^{25}$ 118 (c 1.0 ; Aceton)

Elementaranalyse : $C_7H_{12}O_6S$ (224.24)
Ber. : C (37.50), H (5.40), S (14.30)
Gef. : C (37.41), H (5.59), S (13.7 )

b) 2-(9-Adenyl)-2-desoxy-1.4;3.6-dianhydro-D-glucit :

Das Gemisch aus 112 g (0.5 Mol) 1.4;3.6-Dianhydro-D-mannit-2-methansulfonat, 86 g (0.55 Mol) Adenin-Natriumsalz und 1 500 ml wasserfr. Dimethylsulfoxid wird 1 Tag bei 100 °C gerührt. Unter vermindertem Druck destilliert man das Lösungsmittel soweit wie möglich ab, versetzt den Rückstand mit 4.5 Litern Wasser und extrahiert 2 Tage in 5 l-Rotationsperforator (Normag) kontinuierlich mit Chloroform. Aus der Chloroformphase kristallisiert das Rohprodukt aus, das nach Umkristallisation aus Aethanol/Wasser 6.9 g (26.2 mmol) Reinprodukt ergibt. Das Filtrat wird mit der Wasserphase der Perforation vereinigt und auf ca. 100 ml konzentriert. Es kristallisieren weitere 21.3 g (80.9 mmol) Produkt aus.

Gesamtausbeute : 28.2 g (107 mmol).
Schmp. (Aethanol/Wasser) : 265-7° ;
$[\alpha]_D^{25}$ 20.8 (c 1.0 ; Wasser)
Elementaranalyse : $C_{11}H_{13}N_5O_3$ (263.25)
Ber. : C (50.19), H (4.98), N (26.20)
Gef. : C (49.97), H (5.04), N (26.80)

Aus der Chloroformphase der Perforation lassen sich ca. 40 % nicht umgesetztes 1.4;3.6-Dianhydro-D-mannit-2-methansulfonat zurückgewinnen.

c) 2-(9-Adenyl)-2-desoxy-1.4;3.6-dianhydro-D-glucit-5-nitrat :

Unter Rühren und Kühlen auf − 20° tropft man zu 60 ml 96 %iger Salpetersäure (d = 1.5 ; 1.37 Mol) die Lösung von 4 g (67 mmol) Harnstoff in 170 ml konz. Schwefelsäure (d = 1.84 ; ca 3 Mol) und anschließend die Lösung von 21 g (80 mmol) 2-(9-Adenyl)-2-desoxy-1.4;3.6 dianhydro-D-glucit in 12 ml Wasser + 30 ml Methansulfonsäure zu und rührt bei − 20° eine Stunde nach. Man gießt die Mischung in 1 Liter Eiswasser und tropft unter Rühren die Lösung von 300 g (7.5 Mol) Natriumhydroxid in 1 Liter Wasser zu. Durch Zugabe von wäßriger Natriumhydrogencarbonat-Lösung vervollständigt man die Neutralisation und extrahiert die Mischung 4 mal mit je 1.5 Litern Chloroform. Die vereinigten Chloroformphasen ergeben nach dem Trocknen über wasserfreiem Natriumsulfat, Filtrieren und Eindampfen unter reduz. Druck 16.8 g (54.5 mmol) Rohbase. Diese wird in das Hydrochlorid übergeführt und ergibt nach Umkristallisation aus Methanol 16.3 g (47.3 mmol) 2-(9-Adenyl)-2-desoxy-1.4;3.6-dianhydro-D-glucit-5-nitrat-Hydrochlorid.

Schmp. (aus Wasser) : 214-5° (Z) ;
$[\alpha]_D^{25}$ 62.6 (c 0.5 ; Wasser)
Elementaranalyse ; $C_{11}H_{12}N_6O_5$ x HCl (344.71)
Ber. : C (38.33), H (3.80), N (24.38), Cl (10.28)
Gef. : C (38.21), H (3.59), N (24.46), Cl (10.3 )

## Beispiel Nr. 16

5-{6-[3-(4-Chlorphenyl)-propylamino]-purin-9-yl}-5-desoxy-1.4;3.6-dianhydro-L-idit-2-nitrat

a) 5-{6-[3-Chlorphenyl)-propylamino]-purin-9-yl}-5-desoxy-1.4;3.6-dianhydro-L-idit :

Herstellung analog Beispiel 14 a durch Umsetzung von 3-(4-Chlorphenyl)-1-aminopropan mit 5-(6-Methylmercapto-purin-9-yl)-5-desoxy-1.4;3.6-dianhydro-L-idit. Nach Ueberführung in das Hydrochlorid und Umkristallisation aus 96 %igem Aethanol erhält man in 80 % Ausbeute das reine Produkt in Form des Hydrochlorids-Halbhydrates mit Schmp. 116-120° und $[\alpha]_D^{25}$ 39 (c 0.4 ; Aethanol).

Elementaranalyse : $C_{20}H_{22}ClN_5O_3$ x HCl x 0.5 $H_2O$ (461.35)
Ber. : C (52.07), H (5.24), N (15.18), Cl (15.37)
Gef. : C (52.34), H (5.20), N (15.15), Cl (15.5 )

b) Veresterung mit Salpetersäure :

Analog Beispiel 14 b. Nach Ueberführung in das Hydrochlorid und zweimaliger Umkristallisation aus Methanol/Wasser erhält man in 24 %iger Ausbeute reines 5-{6-[3-(4-Chlorphenyl)-propylamino]-purin-9-yl}-5-desoxy-1.4;3.6-dianhydro-L-idit-2-nitrat-Hydrochlorid mit

Schmp. 183-185° (Z) und $[\alpha]_D^{25}$ 42.5 (c. 0.2 ; Aethanol).
Elementaranalyse : $C_{20}H_{21}ClN_6O_5$ x HCl (497.35)
Ber. : C (48.30), H (4.46), N (16.90), Cl (14.26)
Gef. : C (48.38), H (4.46), N (16.72), Cl (14.1 )

Als Vergleichsverbindungen wurden bei der Untersuchung der pharmakologischen Eigenschaften der erfindungsgemäßen Verbindungen stets die im Handel erhältlichen Verbindungen Isosorbiddinitrat (ISDN) und Isosorbidmononitrat (ISMN) verwendet, wobei es sich bei ISMN um das 1.4;3.6-Dianhydro-D-glucit-2-nitrat handelt.

Die koronardurchflußsteigernde Wirksamkeit der erfindungsgemäßen Verbindungen wurde an isolierten Meerschweinchenherzen (isolierte Herzen nach Langendorff, Methode nach Bunger et al. ; Pflüger's Archiv, 353, 317-325 (1975)) ermittelt. Nach Erreichen des stationären Zustands in der 30. Minute wurden die Herzen mit je 50 ml Tyrodelösung mit einem Gehalt an Prüfsubstanz von jeweils 25 µg/ml perfundiert. Jede Prüfsubstanz wurde an 3-6 Herzen geprüft.

Gemessen wurde jeweils die Inotropie, der Durchfluß und die Frequenz, wobei die in der Tabelle 1 angegebenen Werte Mittelwerte der prozentualen Aenderungen gegenüber dem Vorlauf sind. Der Vergleich der gemessenen Werte zeigt, daß die koronardurchflußsteigernde Wirksamkeit der erfindungsgemäßen Verbindungen größer als diejenige von ISMN ist ; die koronardurchflußsteigernde Wirksamkeit mancher Verbindungen übertrifft sogar diejenige des ISDN.

Tabelle I

Versuche an Langendorff — Herzen

| Substanz Verbindung gem. Beispiel | Dosis [µg/ml] | Inotropie | Durchfluß | Frequenz | Bemerkungen |
|---|---|---|---|---|---|
| 1c oder 2c | 25 | - 8,97 | + 92,13 | + 4,92 | |
| 3c | 25 | - 4,74 | + 39,88 | + 0,60 | |
| 4b | 25 | - 7,49 | + 103,37 | - 4,92 | |
| 5b | 25 | - 6,10 | + 66,97 | - 1,54 | |
| 6b | 25 | - 12,96 | + 23,94 | + 0,15 | |
| 9b | 25 | - 5,15 | + 35,93 | - 7,58 | |
| 12b | 25 | 0 | + 11,43 | + 7,84 | |
| ISDN | 25 | - 8,11 | + 91,54 | + 2,00 | Vergleichs-verbindungen |
| ISMN | 25 | - 2,67 | + 9,11 | - 0,51 | |

Die spasmolytische Wirksamkeit der erfindungsgemäßen Verbindungen wurde an isolierten Ratten-Aorta-Präparaten mit Noradrenalin-induzierten und Kaliumchlorid-induzierten Kontraktionen bestimmt (Methode nach Wende und Peiper, Pflüger's Archiv *320*, 133-141 (1970) ; und Towart und Stoepel, Naunyn Schmiedeberg's Archives of Pharmacology ; Suppl. Vol. *308*, R 18 (1979)).

In Tabelle II sind die Konzentrationen der Prüfsubstanzen angegeben, die für 50 % Hemmung des Spasmus notwendig sind ($ED_{50}$-Werte). Die spasmolytischen Wirksamkeiten der erfindungsgemäßen Verbindungen sind ganz überwiegend besser als diejenigen von ISMN und ISDN, besonders wenn man das pharmakologisch wichtige Verhältnis der effektiven Dosen beim Noradrenalinspasmus und Kaliumchloridspasmus berücksichtigt.

Tabelle II

$ED_{50}$-Werte für spasmolytische Wirkungen

| Substanz Verbindung gem. Beispiel | Noradrenalinspasmus [Mol/l] | Kaliumchloridspasmus [Mol/l] |
|---|---|---|
| 1c oder 2c | $1,35 \times 10^{-7}$ | $2,80 \times 10^{-6}$ |
| 3c | $4,80 \times 10^{-7}$ | $4,60 \times 10^{-6}$ |
| 4b | $2,20 \times 10^{-7}$ | $2,90 \times 10^{-5}$ |
| 5b | $3,80 \times 10^{-7}$ | $1,30 \times 10^{-5}$ |
| 6b | $5,40 \times 10^{-7}$ | $4,07 \times 10^{-6}$ |
| 7b | $2,40 \times 10^{-7}$ | $2,90 \times 10^{-6}$ |
| 8b | $2,90 \times 10^{-7}$ | $3,00 \times 10^{-6}$ |
| 9b | $2,65 \times 10^{-6}$ | $2,50 \times 10^{-5}$ |
| 10 | $3,02 \times 10^{-7}$ | $6,50 \times 10^{-6}$ |
| 12b | $3,20 \times 10^{-6}$ | $4,20 \times 10^{-6}$ |
| 13b | $1,68 \times 10^{-8}$ | $2,40 \times 10^{-6}$ |
| ISDN (Vergleich) | $1,30 \times 10^{-6}$ | $3,10 \times 10^{-6}$ |
| ISMN (Vergleich) | $1,60 \times 10^{-5}$ | $2,40 \times 10^{-6}$ |

Die blutdrucksenkende Wirksamkeit der erfindungsgemäßen Verbindungen wurde an narkotisierten Meerschweinchen nach intravenöser Applikation gemessen. Die in Tabelle III wiedergegebenen Werte zeigen, daß die erfindungsgemäßen Verbindungen wesentlich wirksamer als ISMN sind, wobei das Adenyl-Derivat des 1.4;3.6-Dianhydro-idit-mononitrats (Verbindung gemäß Beispiel 1c/2c) sogar wirksamer ist als ISDN.

Die Herzkreislaufwirksamkeit der erfindungsgemäßen Verbindungen wurde an mischrassigen Katzen von 2,5 bis 3,5 kg Körpergewicht bei intraduodenaler Applikation bestimmt. Die Tiere wurden mit einem Gemisch von Chloralose-Urethan narkotisiert (1,2 g/kg Urethan + 40 mg/kg Chloralose i. p. verabreicht). Sie atmeten spontan durch eine Tracheakanule. Die A. Carotis sinistra wurde benutzt, um ein Katheter-Tip-Manometer in die linke Herzkammer zu legen. Die V. Jugularis diente zu Injektionszwecken. Ueber die A. Femoralis dextra wurde ein Katheter bis in die Aorta descendans vorgeschoben und an einen Druckaufnehmer (Statham P 23Db) angeschlossen. Die Herzfrequenz wurde mit einem Pulsfrequenzmesser (Fa. Hugo Sachs Elektronik) aus dem linksventrikulären Drucksignal registriert. Durch eine Laparotomie wurde eine Duodenalschlinge freigelegt. Die zu prüfenden Substanzen wurden direkt ins Lumen injiziert. Wie sich aus den in der Tabelle IV angegebenen Werten ergibt, ist das Ausmaß der Blutdrucksenkung bei den erfindungsgemäßen Verbindungen deutlich höher und vor allem länger anhaltend als bei den Vergleichsverbindungen ISMN und ISDN. Dies bedeutet, daß die erfindungsgemäßen Verbindungen herzschonend sind und eine geringere negativ-inotrope Wirkung besitzen als die Vergleichsverbindungen.

Die kreislaufpharmakologische Wirksamkeit der erfindungsgemäßen Verbindungen wurde am narkotisierten Hund bei intravenöser Applikation bestimmt. Die Untersuchungen wurden an gemischtrassigen Hunden beiderlei Geschlechtes, Körpergewicht 20.5 bis 29 kg durchgeführt. Nach einer zwölfstündigen Nüchternperiode erhielten die Tiere am Morgen des Versuchstages 2 mg/kg Morphin s. c. Etwa 30 Minuten danach wurde die Narkose mit 25 mg/kg Nembutal i. v. eingeleitet und durch kontinuierliche Infusion mit 5 mg/kg/h fortgeführt. Die Tiere wurden durch einen Trachealtubus mit einem $N_2O/O_2$-Gemisch (2 : 1) konstant beatmet. Der $CO_2$-Gehalt der Ausatemluft wurde kontinuierlich, der pH-Wert des Blutes etwa alle 20 Minuten gemessen.

Die Arteria saphena dextra wurde zur fortlaufenden Messung des arteriellen Blutdruckes freigelegt,

die Arteria femoralis sinistra zur Anlegung eines Statham-Flußmeßkopfes. Von der Arteria brachialis sinistra wurde eine Thermosonde in den Aortabogen vorgeschoben ; sie diente der Messung des Herzminutenvolumens mittels der Thermodilutionsmethode. Unter Röntgenkontrolle wurde durch die Arteria carotis dextra ein Katheter-Tip-Manometer in den linken Ventrikel eingebracht und damit der Linksventrikulär-enddiastolische Druck ermittelt. Von der rechten Vena jugularis wurde ein doppellumiger Katheter in die Arteria pulmonalis eingebracht ; über ein Lumen wurde eisgekühlte isotone NaCl-Lösung zur Bestimmung des Herzminutenvolumens eingespritzt, über das andere der Blutdruck in der Arteria pulmonalis gemessen. Von einer Vene des linken Vorderfußes wurde ein Katheter in den rechten Vorhof zur Druckmessung eingeschoben. Mit dem Differentiator wurde aus dem Druck im linken Ventrikel die Druckanstiegsgeschwindigkeit (« dp/dt ») als Maß für die Kontraktilität errechnet. Nach Beendigung der Präparation wurden die Tiere mit 500 I. E./kg Heparin antikoaguliert.

Nach einer Stabilisationsperiode von etwa 30 Minuten wurde das Herzminutenvolumen bestimmt. Dann erhielten je vier Tiere 1 mg/kg ISDN bzw. 1c/2c i. v. verabreicht. Die Parameter Herzfrequenz, arterieller Blutdruck, Pulmonalisdruck, Druck im rechten Vorhof, linksventrikulärer Druck, dp/dt und mittlerer Femoralisfluß wurden mit einem Beckmann-8-Kanal-Schreiber fortlaufend registriert. Weitere Messungen erfolgten nach der 2., 5., 7., 10., 20., 25., 30., 40., 50., 60., 120., 150., und 180. Minute. Alle Flußgrößen und daraus abgeleiteten Größen wurden auf ein Körpergewicht von 20 kg bezogen. Herzleistungen und Widerstände wurden in der üblichen Art errechnet.

Wie sich aus den in den Tabellen V (Kreislaufpharmakologische Wirkungen der Vergleichsverbindung ISDN) und VI (Kreislaufpharmakologische Wirkungen der erfindungsgemäßen Verbindung 1c/2c) angegebenen Werten ergibt, ist die Wirksamkeit der erfindungsgemäßen Verbindung besser als diejenige der Vergleichsverbindung ISDN, insbesondere was die Senkung des enddiastolischen Druckes im linken Ventrikel und die langfristige Herabsetzung des Druckes in der A. Pulmonalis betrifft.

Legende zu den Tabellen V und VI :

Kreislaufpharmakologische Wirkungen von ISDN (Tab. V) und 1c/2c (Tab. VI) am Hund nach intravenöser Applikation (1 mg/kg). Mittelwerte und Standardfehler aus je 4 Tieren vor der Applikation (v) bzw. verschiedene Zeiten (in min.) nach der Applikation. HF : Herzfrequenz ($min^{-1}$) ; $AP_{syst.}$, $AP_{\underline{diast.}}$ : Systolischer und diastolischer Aortendruck (mm Hg) ; PRV : Druck im rechten Vorhof (mm Hg) ; $\overline{PAP}$ : Mittlerer Druck in der A. Pulmonalis (mm Hg) ; LVEDP : Enddiastolischer Druck im linken Ventrikel (mm Hg) ; dp/dt : Druckanstiegsgeschwindigkeit im linken Ventrikel (mm Hg/sec) ; HLC : Herzleistung links, bezogen auf 20 kg Körpergewicht (W) ; $HL_R$ : Herzleistung rechts, bezogen auf 20 kg Körpergewicht (mW) ; SV : Schlagvolumen, bezogen auf 20 kg Körpergewicht (ml) ; $BF_{Fem}$ : Blutfluß in der A. Femoralis, bezogen auf 20 kg Körpergewicht (ml/min) ; $W_{Fem}$ : Femoraliswiderstand, bezogen auf 20 kg KG (KU) ; HZV : Herzminutenvolumen, bezogen auf 20 kg KG (1/min) ; $W_{AP}$ : Pulmonaliswiderstand, bezogen auf 20 kg KG (U) ; $W_{TP}$ : Gesamter peripherer Widerstand, bezogen auf 20 kg Körpergewicht (U).

(Siehe Tabellen, Seite 20 ff.)

Tabelle III

Blutdruckversuche an Meerschweinchen

| Substanz | Dosis mg/kg | Blutdruck vorher mm Hg | Blutdruck nachher mm Hg | Δ mm Hg |
|---|---|---|---|---|
| ISDN | 0,25 | 68,70 ± 2,30 | 57,00 ± 2,10 | - 11,70 |
| | 1,00 | 66,00 ± 3,50 | 46,30 ± 0,90 | - 19,70 |
| | 2,50 | 66,70 ± 1,70 | 37,70 ± 0,90 | - 29,00 |
| ISMN | 0,25 | 57,60 ± 3,10 | 53,90 ± 3,10 | - 3,70 |
| | 1,00 | 54,70 ± 3,80 | 48,40 ± 3,10 | - 6,30 |
| | 2,50 | 52,30 ± 4,80 | 41,40 ± 3,90 | - 10,90 |
| 1c/2c | 0,25 | 68,25 ± 2,50 | 50,50 ± 2,33 | - 17,75 |
| | 1,00 | 69,50 ± 2,90 | 41,50 ± 3,97 | - 28,00 |
| | 2,50 | 70,00 ± 2,27 | 38,75 ± 2,66 | - 31,25 |
| 3c | 0,25 | 79,50 ± 2,50 | 79,25 ± 2,56 | - 0,25 |
| | 1,00 | 79,50 ± 2,66 | 71,50 ± 3,97 | - 8,00 |
| | 2,50 | 77,00 ± 2,80 | 61,50 ± 3,23 | - 15,50 |
| 4b | 0,25 | 73,75 ± 2,14 | 63,25 ± 4,23 | - 10,50 |
| | 1,00 | 74,00 ± 1,91 | 59,00 ± 1,47 | - 15,00 |
| | 2,50 | 72,00 ± 2,45 | 43,50 ± 2,50 | - 28,50 |
| 5b | 0,25 | 76,50 ± 2,66 | 71,00 ± 5,04 | - 5,50 |
| | 1,00 | 78,50 ± 1,70 | 65,75 ± 3,75 | - 12,75 |
| | 2,50 | 76,25 ± 2,59 | 51,00 ± 3,13 | - 25,25 |
| 6b | 0,25 | 65,00 ± 4,02 | 51,50 ± 4,94 | - 13,50 |
| | 1,00 | 65,50 ± 2,36 | 44,75 ± 2,84 | - 20,75 |
| | 2,50 | 62,00 ± 2,04 | 37,25 ± 1,11 | - 24,75 |
| 9b | 0,25 | 72,50 ± 6,20 | 70,25 ± 8,39 | - 2,25 |
| | 1,00 | 73,25 ± 6,24 | 69,75 ± 7,44 | - 3,50 |
| | 2,50 | 72,50 ± 5,24 | 59,75 ± 4,82 | - 12,75 |

Tabelle IV

| Substanz + Dosis | Zeit (min) nach Appl. | Frequenz $min^{-1}$ | Blutdruck mm Hg | Δ | dp/dt mm sec. | Δ |
|---|---|---|---|---|---|---|
| ISDN 5 mg/kg | 0 | 174,3 ± 9,3 | 104,3 ± 7,7 | - 13,9 | 8800 ± 831 | - 800 |
| | 10 | 179,3 ± 7,7 | 90,4 ± 12,5 | - 13,1 | 8000 ± 1097 | -1133 |
| | 30 | 177,7 ± 7,7 | 91,2 ± 10,0 | - 10,1 | 7667 ± 807 | -1167 |
| | 60 | 175,0 ± 8,6 | 94,2 ± 9,2 | - 9,3 | 7633 ± 743 | -1750 |
| | 120 | 166,7 ± 9,4 | 95,0 ± 9,6 | | 7050 ± 661 | |
| 1c/2c 5 mg/kg | 0 | 171,6 ± 6,2 | 124,2 ± 8,3 | - 15,6 | 7220 ± 611 | + 280 |
| | 10 | 180,4 ± 4,3 | 108,6 ± 8,5 | - 16,4 | 7500 ± 522 | - 260 |
| | 30 | 177,2 ± 5,4 | 107,8 ± 8,4 | - 17,6 | 6960 ± 564 | - 120 |
| | 60 | 177,6 ± 5,1 | 106,6 ± 7,8 | - 18,0 | 7100 ± 721 | - 220 |
| | 120 | 178,8 ± 4,4 | 106,2 ± 5,6 | | 7000 ± 1026 | |

Legende : Herzfrequenz, Blutdruck und Inotropie zu verschiedenen Zeiten nach intraduodenaler Verabreichung von ISDN oder Substanz 1c/2c an Katzen.
Mittelwerte ± Standardfehler aus je 6 Tieren

Tabelle V : ISDN (Vergleich)

| | v | 2 | 5 | 7 | 10 | 15 | 20 | 25 |
|---|---|---|---|---|---|---|---|---|
| HF | 109,5 ± 7,1 | 143,7 ± 5,1 | 133,5 ±11,9 | 128,7 ±11,1 | 121,7 ±10,6 | 118,2 ± 9,8 | 112,5 ± 8,8 | 112,0 ± 8,5 |
| $AP_{syst}$ | 166,9 ± 4,9 | 145,7 ± 7,9 | 152,9 ± 6,4 | 152,9 ± 6,5 | 152,9 ± 5,6 | 154,8 ± 4,7 | 154,8 ± 5,6 | 156,6 ± 4,7 |
| $AP_{diast}$ | 95,6 ±10,8 | 99,4 ± 9,7 | 102,2 ± 7,7 | 100,3 ± 8,0 | 98,4 ± 9,0 | 98,4 ± 9,0 | 96,6 ± 7,4 | 98,4 ± 7,2 |
| PRV | 2,06 ± 0,5 | 1,12 ± 0,6 | 1,72 ± 0,7 | 1,78 ± 0,7 | 1,87 ± 0,6 | 1,97 ± 0,7 | 2,15 ± 0,7 | 2,38 ± 0,8 |
| $\overline{PAP}$ | 14,3 ± 1,2 | 11,1 ± 0,7 | 11,5 ± 0,8 | 12,6 ± 1,4 | 12,6 ± 1,4 | 12,7 ± 1,6 | 12,6 ± 1,3 | 13,3 ± 1,5 |
| LVEDP | 7,69 ± 1,3 | 4,97 ± 1,1 | 5,81 ± 0,8 | 6,0 ± 0,9 | 6,28 ± 0,8 | 6,47 ± 0,7 | 6,75 ± 0,7 | 7,03 ± 0,7 |
| dp/dt | 2268,7 ±271,1 | 2868,7 ±370,4 | 2606,2 ±30,1 | 2587,5 ±291,0 | 2550,0 ±302,2 | 2512,5 ±288,0 | 2418,7 ± 281,2 | 2456,2 ±295,9 |
| $HL_L$ | 0,76 ±0,13 | 0,94 ±0,15 | 0,95 ±0,17 | 0,85 ±0,14 | 0,84 ±0,15 | 0,79 ±0,14 | 0,78 ±0,13 | 0,76 ±0,13 |
| $HL_R$ | 85,1 ±19,8 | 85,3 ±11,3 | 81,4 ±13,9 | 83,0 ±18,3 | 82,4 ±17,9 | 78,9 ±21,3 | 75,3 ±16,3 | 76,3 ±18,2 |
| SV | 27,7 ±2,8 | 26,3 ±1,8 | 27,4 ±2,1 | 25,9 ±1,5 | 27,4 ±1,9 | 26,8 ±2,3 | 27,9 ±2,3 | 26,8 ±2,1 |
| $BF_{Fem}$ | 44,9 ±11,7 | 31,2 ±6,5 | 35,4 ±7,7 | 37,3 ±7,1 | 38,4 ±6,6 | 40,9 ±8,6 | 40,9 ±7,2 | 41,9 ±8,5 |
| $W_{Fem}$ | 0,39 ±0,1 | 0,55 ±0,1 | 0,49 ±0,1 | 0,45 ±0,1 | 0,42 ±0,05 | 0,41 ±0,1 | 0,40 ±0,05 | 0,40 ±0,1 |
| HZV | 3,03 ±0,4 | 3,8 ±0,4 | 3,7 ±0,5 | 3,3 ±0,4 | 3,4 ±0,4 | 3,2 ±0,4 | 3,1 ±0,4 | 3,0 ±0,4 |
| $W_{AP}$ | 0,31 ±0,05 | 0,23 ±0,05 | 0,22 ±0,04 | 0,27 ±0,03 | 0,26 ±0,03 | 0,26 ±0,03 | 0,25 ±0,02 | 0,27 ±0,02 |
| $W_{TP}$ | 5,27 ±0,4 | 4,02 ±0,1 | 4,39 ±0,4 | 4,72 ±0,3 | 4,66 ±0,3 | 4,96 ±0,3 | 4,92 ±0,3 | 5,22 ±0,4 |

Tabelle V (Fortsetzung) : ISDN (Vergleich)

| | 30 | 40 | 50 | 60 | 90 | 120 | 150 | 180 |
|---|---|---|---|---|---|---|---|---|
| HF | 110,0 ±17,5 | 108,0 ± 9,8 | 109,2 ±21,2 | 107,5 ±10,1 | 109,5 ± 9,9 | 104,2 ± 7,7 | 103,7 ± 6,9 | 103,7 ± 5,9 |
| $AP_{syst}$ | 155,6 ± 3,2 | 158,4 ± 4,9 | 157,5 ± 5,1 | 159,4 ± 5,8 | 163,2 ± 5,8 | 161,2 ± 6,6 | 161,2 ± 4,7 | 161,2 ± 4,8 |
| $AP_{diast}$ | 98,1 ± 5,9 | 101,2 ± 6,5 | 101,2 ± 6,5 | 101,2 ± 6,5 | 99,4 ±5,6 | 97,5 ±5,5 | 95,6 ± 4,5 | 95,6 ± 5,6 |
| PRV | 2,34 ±0,7 | 2,34 ±0,7 | 2,29 ±0,6 | 2,53 ±0,7 | 2,53 ±0,7 | 2,81 ±0,8 | 3,o ±0,9 | 2,81 ±0,8 |
| $\overline{PAP}$ | 13,5 ±1,5 | 14,0 ±1,5 | 14,0 ±1,6 | 14,1 ±1,6 | 14,6 ±1,8 | 15,0 ±2,0 | 14,6 ±1,5 | 15,5 ±1,6 |
| LVEDP | 7,22 ±0,6 | 7,60 ±0,7 | 7,60 ±0,7 | 7,77 ±0,7 | 7,98 ±0,9 | 8,34 ±1,0 | 7,78 ±1,0 | 7,60 ±1,2 |
| dp/dt | 2390,6 ±284,9 | 2343,7 ±281,2 | 2306,2 ±269,2 | 2250,0 ±250,6 | 2231,2 ±251,2 | 2118,7 ±221,5 | 2025,0 ±181,1 | 2043,7 ±206,2 |
| $HL_L$ | 0,76 ±0,10 | 0,76 ±0,13 | 0,77 ±0,13 | 0,78 ±0,14 | 0,77 ±0,12 | 0,72 ±0,10 | 0,74 ±0,10 | 0,79 ±0,12 |
| $HL_R$ | 78,6 ±16,5 | 80,5 ±17,2 | 81,1 ±18,6 | 81,9 ±20,1 | 85,4 ±20,5 | 81,5 ±18,9 | 79,7 ±17,2 | 92,4 ±18,7 |
| SV | 27,8 ±2,1 | 27,7 ±1,9 | 27,4 ±1,6 | 28,2 ±1,6 | 27,7 ±1,9 | 28,1 ±1,8 | 28,6 ±1,6 | 30,6 ±1,9 |
| $BF_{Fem}$ | 41,5 ±8,1 | 42,9 ±8,2 | 43,9 ±9,2 | 42,3 ±8,9 | 44,4 ±8,2 | 49,3 ±7,7 | 51,3 ±9,8 | 53,7 ±10,8 |
| $W_{Fem}$ | 0,40 ±0,1 | 0,40 ±0,05 | 0,39 ±0,1 | 0,41 ±0,1 | 0,38 ±0,04 | 0,33 ±0,03 | 0,32 ±0,05 | 0,32 ±0,06 |
| HZV | 3,0 ±0,3 | 3,0 ±0,3 | 3,0 ±0,4 | 3,0 ±0,4 | 3,0 ±0,4 | 2,9 ±0,2 | 3,0 ±0,3 | 3,2 ±0,3 |
| $W_{AP}$ | 0,27 ±0,03 | 0,28 ±0,04 | 0,27 ±0,04 | 0,26 ±0,03 | 0,27 ±0,06 | 0,28 ±0,08 | 0,29 ±0,05 | 0,31 ±0,05 |
| $W_{TP}$ | 5,13 ±0,4 | 5,41 ±0,5 | 5,38 ±0,5 | 5,32 ±0,5 | 5,35 ±0,6 | 5,36 ±0,3 | 5,22 ±0,3 | 4,89 ±0,3 |

Tabelle VI : Verbindung gem. Beispiel 1c/2c

| | v | 2 | 5 | 7 | 10 | 15 | 20 | 25 |
|---|---|---|---|---|---|---|---|---|
| HF | 97,5 ±13,0 | 158,7 ±20,7 | 148,7 ±22,1 | 146,7 ±22,5 | 141,2 ±22,5 | 133,0 ±21,9 | 129,7 ±19,6 | 125,7 ±19,1 |
| $AP_{syst}$ | 150,9 ± 8,4 | 115,3 ±13,8 | 120,9 ±13,3 | 121,9 ±11,6 | 120,9 ±10,6 | 120,4 ± 9,3 | 121,5 ± 8,7 | 122,8 ± 8,8 |
| $AP_{diast}$ | 88,1 ±7,7 | 74,1 ±10,0 | 82,5 ±9,4 | 83,4 ±9,4 | 83,4 ±8,4 | 80,6 ±7,7 | 79,1 ±6,7 | 80,6 ±6,6 |
| PRV | 3,94 ±0,19 | 2,06 ±0,45 | 2,81 ±0,57 | 2,81 ±0,45 | 2,95 ±0,50 | 3,24 ±0,54 | 3,30 ±0,52 | 3,28 ±0,41 |
| $\overline{PAP}$ | 15,8 ±1,4 | 15,6 ±2,1 | 13,7 ±1,4 | 14,1 ±1,6 | 14,2 ±1,6 | 14,0 ±1,9 | 13,8 ±1,7 | 14,0 ±1,8 |
| LVEDP | 5,53 ±1,3 | 2,62 ±0,6 | 2,91 ±0,8 | 2,91 ±0,8 | 3,20 ±0,8 | 3,37 ±0,8 | 3,56 ±0,9 | 4,03 ±1,0 |
| dp/dt | 2081,2 ±253,2 | 2606,2 ±345,5 | 2343,7 ±251,3 | 2362,5 ±255,2 | 2268,7 ±309,8 | 2250,0 ±336,8 | 2231.2 ±330,3 | 2175,0 ±310,7 |
| $HL_L$ | 0,83 ±0,08 | 0,75 ±0,13 | 0,74 ±0,08 | 0,72 ±0,06 | 0,72 ±0,06 | 0,72 ±0,06 | 0,72 ±0,05 | 0,74 ±0,05 |
| $HL_R$ | 96,0 ±15,4 | 115,8 ±18,1 | 87,6 ±5,1 | 89,3 ±16,9 | 88,8 ±17,6 | 87,3 ±20,2 | 87,7 ±20,0 | 90,0 ±20,2 |
| SV | 38,3 ±3,6 | 25,6 ±2,6 | 26,0 ±4,0 | 25,6 ±4,1 | 26,4 ±3,7 | 29,3 ±5,0 | 30,0 ±4,4 | 31,3 ±4,6 |
| $BF_{Fem}$ | 53,4 ±7,5 | 35,3 ±4,0 | 36,6 ±4,0 | 38,6 ±4,2 | 39,5 ±4,3 | 39,9 ±5,2 | 41,2 ±5,3 | 42,2 ±5,4 |
| $W_{Fem}$ | 0,28 ±0,04 | 0,35 ±0,08 | 0,36 ±0,06 | 0,34 ±0,06 | 0,33 ±0,05 | 0,32 ±0,05 | 0,31 ±0,04 | 0,31 ±0,05 |
| HZV | 3,61 ±0,25 | 3,91 ±0,27 | 3,61 ±0,08 | 3,51 ±0,16 | 3,50 ±0,11 | 3,61 ±0,21 | 3,66 ±0,18 | 3,70 ±0,18 |
| $W_{AP}$ | 0,38 ±0,03 | 0,45 ±0,09 | 0,40 ±0,07 | 0,42 ±0,06 | 0,42 ±0,06 | 0,40 ±0,08 | 0,37 ±0,07 | 0,36 ±0,08 |
| $W_{TP}$ | 3,94 ±0,3 | 2,93 ±0,3 | 3,42 ±0,4 | 3,59 ±0,5 | 3,57 ±0,4 | 3,41 ±0,4 | 3,32 ±0,4 | 3,33 ±0,4 |

Tabelle VI (Fortsetzung) : Verbindung gem. Beispiel 1c/2c

| | 30 | 40 | 50 | 60 | 90 | 120 | 150 | 180 |
|---|---|---|---|---|---|---|---|---|
| HF | 122,7<br>±18,5 | 120,7<br>±17,4 | 118,7<br>±18,3 | 114,2<br>±20,1 | 107,5<br>±17,5 | 98,7<br>±11,7 | 97,5<br>±11,1 | 95,5<br>±8,5 |
| $AP_{syst}$ | 122,8<br>± 8,8 | 126,6<br>± 8,8 | 131,2<br>± 8,9 | 133,1<br>± 9,8 | 136,9<br>±10,9 | 143,4<br>± 7,2 | 147,2<br>± 7,1 | 143,4<br>± 6,2 |
| $AP_{diast}$ | 79,7<br>±7,1 | 81,0<br>±6,8 | 83,4<br>±6,2 | 87,6<br>±6,2 | 87,6<br>±6,1 | 93,7<br>±3,4 | 94,7<br>± 3,9 | 91,9<br>± 3,9 |
| PRV | 3,52<br>±0,61 | 3,37<br>±0,59 | 3,43<br>±0,55 | 3,37<br>±0,59 | 3,85<br>±0,52 | 3,94<br>±0,49 | 4,03<br>±0,39 | 4,12<br>±0,31 |
| $\overline{PAP}$ | 14,1<br>±1,8 | 14,1<br>±1,8 | 13,9<br>±,9 | 14,1<br>±2,0 | 14,5<br>±2,1 | 15,1<br>±2,3 | 15,1<br>±1,5 | 14,9<br>±1,3 |
| LVEDP | 4,31<br>±1,1 | 4,41<br>±1,2 | 4,69<br>±1,2 | 4,78<br>±1,3 | 5,25<br>±1,4 | 6,37<br>±1,2 | 6,97<br>±1,4 | 7,69<br>±1,3 |
| dp/dt | 2184,4<br>±330,6 | 2137,5<br>±327,6 | 2118,5<br>±315,8 | 2118,5<br>±335,9 | 2081,2<br>±380,4 | 2043,7<br>±402,0 | 1950,0<br>±373,7 | 1593,7<br>±297,4 |
| $HL_L$ | 0,73<br>±0,06 | 0,76<br>±0,05 | 0,75<br>±0,04 | 0,79<br>±0,05 | 0,82<br>±0,04 | 0,89<br>±0,06 | 0,91<br>±0,05 | 0,91<br>±0,06 |
| $HL_R$ | 84,9<br>±17,7 | 89,3<br>±19,6 | 83,6<br>±20,3 | 89,5<br>±21,8 | 91,5<br>±26,7 | 98,7<br>±29,7 | 98,0<br>±21,3 | 98,5<br>±20,0 |
| SV | 31,6<br>±4,5 | 32,7<br>±4,5 | 32,0<br>±4,7 | 35,6<br>±5,8 | 37,1<br>±4,4 | 40,0<br>±2,7 | 41,0<br>±3,3 | 42,6<br>±1,9 |
| $BF_{Fem}$ | 43,9<br>±4,9 | 46,3<br>±4,3 | 50,6<br>±4,5 | 50,4<br>±5,5 | 50,8<br>±6,6 | 58,6<br>±7,9 | 58,2<br>±8,1 | 59,4<br>±6,3 |
| $W_{Fem}$ | 0,29<br>±0,04 | 0,27<br>±0,03 | 0,26<br>±0,02 | 0,26<br>±0,03 | 0,27<br>±0,03 | 0,25<br>±0,03 | 0,26<br>±0,04 | 0,24<br>±0,02 |
| HZV | 3,63<br>±0,05 | 3,72<br>±0,10 | 3,55<br>±0,08 | 3,73<br>±0,03 | 3,77<br>±0,16 | 3,85<br>±0,23 | 3,89<br>±0,18 | 4,03<br>±0,23 |
| $W_{AP}$ | 0,36<br>±0,10 | 0,35<br>±0,10 | 0,34<br>±0,10 | 0,33<br>±0,10 | 0,32<br>±0,09 | 0,30<br>±0,07 | 0,27<br>±0,05 | 0,24<br>±0,03 |
| $W_{TP}$ | 3,32<br>±0,2 | 3,34<br>±0,3 | 3,61<br>±0,3 | 3,46<br>±0,2 | 3,59<br>±0,4 | 3,72<br>±0,3 | 3,73<br>±0,2 | 3,51<br>±0,3 |

Zur orientierenden Prüfung der akuten Toxizität einiger der erfindungsgemäßen Verbindungen wurden diese in physiologischer Kochsalzlösung an weiblichen NMRI-Albinomäusen intravenös in Dosen von 50, 100 und 200 mg/kg appliziert. Die Substanzen wurden an mindestens 3 Tieren pro Dosis gespritzt. Wenn nach der höchsten applizierten Dosis noch kein Tier gestorben war, wurden keine weiteren Substanzdosen geprüft. Im Zweifelsfall wurde die Prüfung an mindestens 3 weiteren Tieren mit der gleichen Dosis wieder holt. Beobachtet wurde die Todesrate innerhalb 24 Stdn. nach der Applikation.

In Tabelle VII sind die ermittelten Todesraten und der daraus abgeschätzte $LD_{50}$-Bereich dargestellt.

Tabelle VII

| Beispiel Nr. | Todeshäufigkeit bei der i.v.Dosis von | | | Geschätzter $LD_{50}$-Bereich (mg/kg) |
|---|---|---|---|---|
| | 200 mg/kg | 100 mg/kg | 50 mg/kg | |
| 3 c | 3/3 | 0/6 | - | 100 - 200 |
| 4 b | 3/3 | 3/3 | 0/3 | 50 - 100 |
| 5 b | 3/3 | 3/6 | 1/11 | 50 - 100 |
| 6 b | 3/3 | 3/3 | 0/3 | 50 - 100 |
| 9 b | 3/3 | 4/6 | - | 50 - 100 |
| 2 c⁺ | 0/6 | - | - | > 200 |

+$LD_{70}$ p. o. Maus = 1 600 mg/kg und $LD_{50}$ i. p. Maus = 540 mg/kg (534.7-545.4 ; nach Lichtfield u. Wilcoxon)

**Ansprüche**

1. Adenyl-desoxy-1.4;3.6-dianhydro-hexit-nitrate der allgemeinen Formel Ia

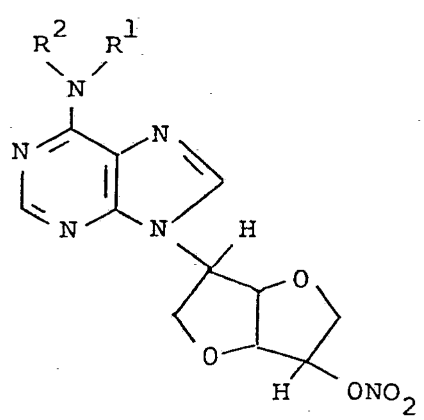

(Ia)

worin $R^1$ und $R^2$ gleich oder verschieden sind und unabhängig voneinander

(a) ein Wasserstoffatom,

(b) eine geradkettige oder verzweigte Alkylgruppe mit 1 bis 7 C-Atomen,

(c) eine ω-Phenylalkylgruppe, wobei die Alkylgruppe 1 bis 7 C-Atome aufweist und wobei der Phenylring in p-Stellung halogensubstituiert sein kann,

bedeuten oder worin

(d) $R^1$ einen der unter (a) bis (c) angegebenen Reste und $R^2$ einen Acylrest einer aliphatischen, gegebenenfalls methylsubstituierten Monocarbonsäure mit 2 bis 7 C-Atomen,

bedeuten oder worin

(e) $R^1$ und $R^2$ gemeinsam mit dem Stickstoffatom, an das sie gebunden sind, den Rest eines cyclischen, nichtaromatischen, gegebenenfalls ein weiteres Heteroatom enthaltenden sekundären Amins darstellen, das ausgewählt ist aus der aus Pyrrolidin, Piperidin, Piperazin, 4-Methylpiperazin und Morpholin bestehenden Gruppe,

sowie deren physiologisch unbedenkliche Säureadditionssalze.

2. Theophyllinyl-desoxy-1.4;3.6-dianhydro-hexit-nitrate der allgemeinen Formel Ib,

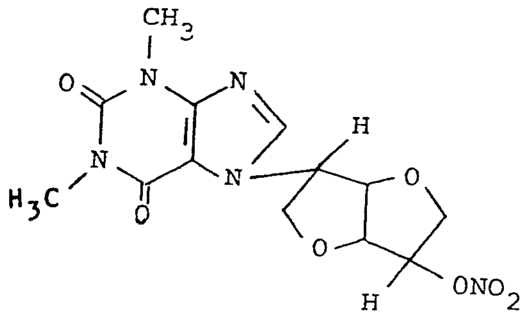

(Ib)

sowie deren physiologisch unbedenkliche Säureadditionssalze.

3. 5-(9-Adenyl)-5-desoxy-1.4;3.6-dianhydro-L-idit-2-nitrate der allgemeinen Formel Ic,

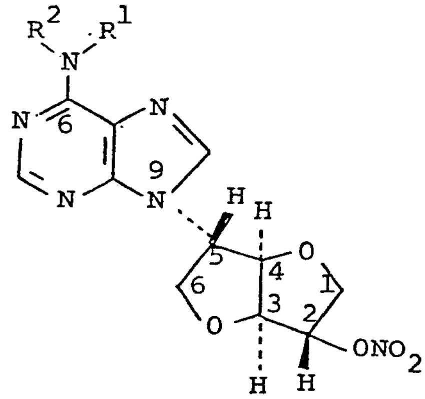

(Ic)

worin $R^1$ und $R^2$ die in Anspruch 1 genannten Bedeutungen besitzen, sowie deren physiologisch unbedenkliche Säureadditionssalze.

4. 2-(9-Adenyl)-2-desoxy-1.4;3.6-dianhydro-D-glucit-5-nitrate der allgemeinen Formel Id,

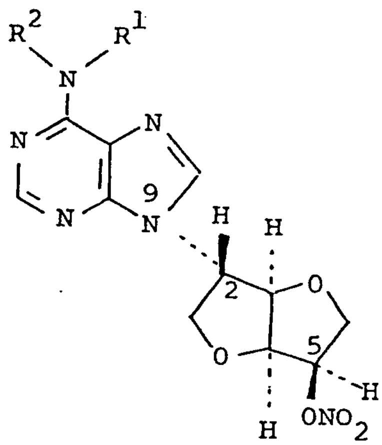

(Id)

worin $R^1$ und $R^2$ die in Anspruch 1 genannten Bedeutungen besitzen, sowie deren physiologisch unbedenkliche Säureadditionssalze.

5. 5-(9-Adenyl)-5-desoxy-1.4;3.6-dianhydro-D-glucit-2-nitrate der allgemeinen Formel Ie,

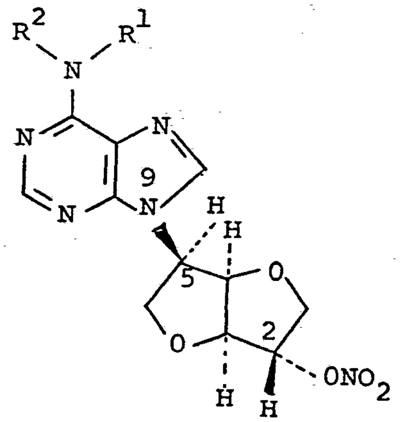

(Ie)

worin $R^1$ und $R^2$ die in Anspruch 1 genannten Bedeutungen besitzen, sowie deren physiologisch unbedenkliche Säureadditionssalze.

6. 5-(7-Theophyllinyl)-5-desoxy-1.4;3.6-dianhydro-L-idit-2-nitrat sowie dessen physiologisch unbedenkliche Säureadditionssalze.

7. 2-(7-Theophyllinyl)-2-desoxy-1.4;3.6-dianhydro-D-Glucit-5-nitrat sowie dessen physiologisch unbedenkliche Säureadditionssalze.

8. 5-(9-Adenyl)-5-desoxy-1.4;3.6-dianhydro-L-idit-2-nitrat.

9. 5-(6-Methylaminopurin-9-yl)-5-desoxy-1.4;3.6-dianhydro-L-idit-2-nitrat.

10. 5-(6-Dimethylaminopurin-9-yl)-5-desoxy-1.4;3.6-dianhydro-L-idit-2-nitrat.

11. 5-(6-Äthylaminopurin-9-yl)-5-desoxy-1.4;3.6-dianhydro-L-idit-2-nitrat.

12. 5-(6-Pyrrolidinopurin-9-yl)-5-desoxy-1.4;3.6-dianhydro-L-idit-2-nitrat.

13. 5-(6-Piperidinopurin-9-yl)-5-desoxy-1.4;3.6-dianhydro-L-idit-2-nitrat.

14. 5-(6-Morpholinopurin-9-yl)-5-desoxy-1.4;3.6-dianhydro-L-idit-2-nitrat.

15. 5-[6-(4-Methylpiperazino)-purin-9-yl]-5-desoxy-1.4;3.6-dianhydro-L-idit-2-nitrat.

16. 5-[9-(6-N-Pivaloyl)-adenyl]-5-desoxy-1.4;3.6-dianhydro-L-idit-2-nitrat.

17. 5-[9-(6-N-Acetyl)-adenyl]-5-desoxy-1.4;3.6-dianhydro-L-idit-2-nitrat.

18. 5-(6-Benzylaminopurin-9-yl)-5-desoxy-1.4;3.6-dianhydro-L-idit-2-nitrat.

19. 5-[6-(3-Phenylpropyl)-aminopurin-9-yl]-5-desoxy-1.4;3.6-dianhydro-L-idit-2-nitrat.

20. 2-(9)Adenyl)-2-desoxy-1.4;3.6-dianhydro-D-glucit-5-nitrat.

21. 5-{6-[3-(4-Chlorphenyl)-propylamino]-purin-9-yl}-5-desoxy-1.4;3.6-dianhydro-L-idit-2-nitrat.

22. Verfahren zur Herstellung der Verbindungen gemäß Anspruch 1, dadurch gekennzeichnet, daß

(A) L-Isoidid, D-Isosorbid oder D-Isomannid mit einem Sulfonsäurechlorid in den entsprechenden Mono-O-acyl-1.4;3.6-dianhydro-hexit übergeführt wird,

(B) welcher mit einem Alkalisalz des Adenins oder eines in 6-N-Stellung substituierten Adenins, des 6-Methylmercaptopurins oder 6-Chlorpurins unter Umkehrung der Konfiguration am Substitutionsort zum entsprechenden Mono-purinyl-1.4;3.6-dianhydrohexit umgesetzt wird,

(C) wonach gegebenenfalls die 6-Methylmercaptogruppe oder das 6-Chloratom des Adenin -Restes mit einem primären oder sekundären Alkylamin mit geradkettiger oder verzweigter Alkylgruppe mit 1 bis 7 C-Atomen oder mit einem ω-Phenylalkylamin mit einer Alkylgruppe mit 1 bis 7 C-Atomen oder mit einem entsprechenden p-halogensubstituierten ω-Phenylalkylamin oder mit einem cyclischen, nicht-aromatischen, gegebenenfalls ein weiteres Heteroatom enthaltenden sekundären Amin umgesetzt wird,

(D) wonach die freie Hydroxylgruppe des entstandenen Adenyl-desoxy-1.4;3.6-dianhydro-hexits in an sich bekannter Weise mit Salpetersäure, Nitriersäure oder mit einem Gemisch aus Salpetersäure und Eisessig/Acetanhydrid zu den entsprechenden Mononitraten der allgemeinen Formeln Ia und Ic bis Ie verestert wird,

(E) wonach gegebenenfalls die 6-Aminogruppe oder die monosubstituierte 6-Aminogruppe des Adenin-Restes in an sich bekannter Weise mit einer aliphatischen, gegebenenfalls methylsubstituierten Monocarbonsäure mit 2 bis 7 C-Atomen zur 6-Acylamino-Gruppe umgesetzt wird.

23. Verfahren zur Herstellung der Verbindungen gemäß Anspruch 3, dadurch gekennzeichnet, daß

(A) D-Isosorbid mit einem Überschuß an Sulfonsäurechlorid, vorzugsweise Methansulfonylchlorid oder Toluolsulfonylchlorid, quantitativ zum entsprechenden Isosorbid-diacylat umgesetzt wird,

(B) welches mit einem Alkalisalz des Adenins oder eines in 6-N-Stellung substituierten Adenins zum entsprechenden 5-(9-Adenyl)-5-desoxy-1.4;3.6-dianhydro-L-idit-2-acylat umgesetzt wird,

(C) wonach die 2-Acylat-Gruppe einer sauren oder alkalischen Hydrolyse unterworfen wird und

(D) der entstehende 5-(9-Adenyl)-5-desoxy-1.4;3.6-dianhydro-L-idit entsprechend dem Verfahren nach Anspruch 22 (D) + (E) weiter aufgearbeitet wird.

24. Verfahren zur Herstellung der Verbindungen gemäß Anspruch 2, dadurch gekennzeichnet, daß gemäß Anspruch 22 gearbeitet wird, aber anstelle eines Alkalisalzes des Adenins, eines 6-N-substituierten Adenins, des 6-Methylmercaptopurins oder 6-Chlorpurins ein Alkalisalz des Theophyllins verwendet wird.

25. Verfahren zur Herstellung der Verbindungen gemäß Anspruch 6, dadurch gekennzeichnet, daß gemäß Anspruch 23 gearbeitet wird, aber anstelle eines Alkalisalzes des Adenins oder eines 6-N-substituierten Adenins ein Alkalisalz des Theophyllins verwendet wird.

26. Verfahren nach einem der Ansprüche 22 oder 24, dadurch gekennzeichnet, daß die Umsetzung des D-Isosorbid-5-acylats bzw. des D-Isomannid-2-acylats mit einem Alkalisalz des Adenins, eines in 6-N-Stellung substituierten Adenins, des 6-Methylmercaptopurins, des 6-Chlorpurins oder des Theophyllins in einem dipolaren aprotischen Lösungsmittel bei einer Temperatur zwischen 80 und 130 °C unter Inertgasatmosphäre erfolgt.

27. Verfahren nach Anspruch 22, dadurch gekennzeichnet, daß die Umsetzung der 6-Methylmercaptogruppe oder des 6-Chloratoms des Purinyl-Restes mit Ammoniak oder einem primären oder sekundären Amin bei einer Temperatur von 100 bis 170 °C und bei einem Druck von 2 bis 20 at unter einer Inertgasatmosphäre erfolgt.

28. Verfahren nach einem der Ansprüche 23 oder 25, dadurch gekennzeichnet, daß die Umsetzung des D-Isosorbiddiacylats mit einem Alkalisalz des Adenins, eines in 6-N-Stellung substituierten Adenins oder des Theophyllins in einem dipolaren aprotischen Lösungsmittel bei einer Temperatur zwischen 80 und 110 °C unter Inertgasatmosphäre erfolgt.

29. Verfahren nach Anspruch 26 oder 28, dadurch gekennzeichnet, daß Dimethylformamid oder Dimethylsulfoxid als dipolare aprotische Lösungsmittel verwendet werden.

30. Verfahren nach Anspruch 27, dadurch gekennzeichnet, daß die Umsetzung in Anwesenheit eines niederen Alkohols als Lösungsmittel erfolgt.

31. Pharmazeutische Zubereitung, enthaltend eine Verbindung gemäß einem der Ansprüche 1 bis 21 sowie übliche Träger und Zusatzstoffe.

## Claims

1. Adenyldesoxy-1.4;3.6-dianhydrohexitol nitrates of the general formula Ia

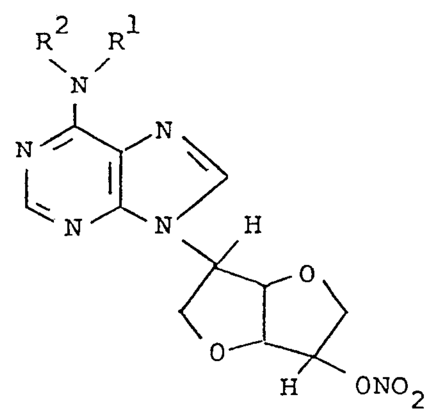

(Ia)

wherein $R^1$ and $R^2$ are the same or different and, independently of one another signify

(a) a hydrogen atom,

(b) a straight-chained or branched alkyl group with 1 to 7 C-atoms,

(c) an ω-phenylalkyl group, whereby the alkyl group has 1 to 7 C-atoms and whereby the phenyl ring can be halogen-substituted in the p-position, or

wherein

(d) $R^1$ signifies one of the residues given under (a) to (c) and $R^2$ an acyl radical of an aliphatic, possibly methyl-substituted monocarboxylic acid with 2 to 7 C-atoms, or

wherein

(e) $R^1$ and $R^2$, together with the nitrogen atom to which they are bound, represent the residue of a cyclic, non-aromatic, secondary amine possibly containing a further hetero atom selected from the group consisting of pyrrolidine, piperidine, piperazine, 4-methylpiperazine, and morpholine,

as well as their physiologically acceptable acid-addition salts.

2. Theophyllinyldesoxy-1.4;3.6-dianhydrohexitol nitrates of the general formula Ib

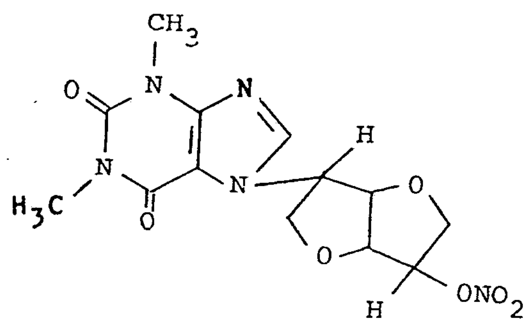

(Ib)

as well as their physiologically acceptable acid-addition salts.

3. 5-(9-Adenyl)-5-desoxy-1.4;3.6-dianhydro-L-iditol 2-nitrates of the general formula Ic

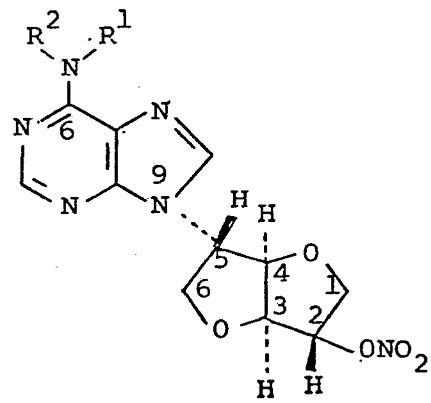

(Ic)

wherein $R^1$ and $R^2$ possess the meanings mentioned in claim 1, as well as their physiologically acceptable acid-addition salts.

4. 2-(9-Adenyl)-2-desoxy-1.4;3.6-dianhydro-D-glucitol 5-nitrates of the general formula Id

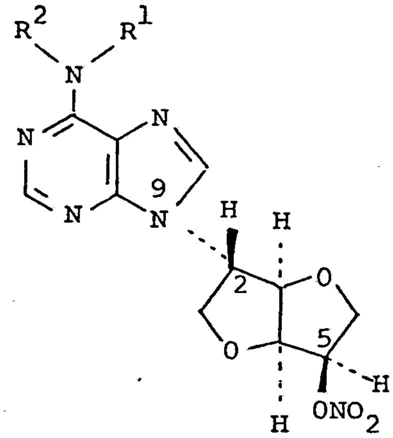

(Id)

wherein $R^1$ and $R^2$ possess the meanings mentioned in claim 1, as well as their physiologically acceptable acid-addition salts.

5. 5-(9-Adenyl)-5-desoxy-1.4;3.6-dianhydro-D-glucitol 2-nitrates of the general formula Ie

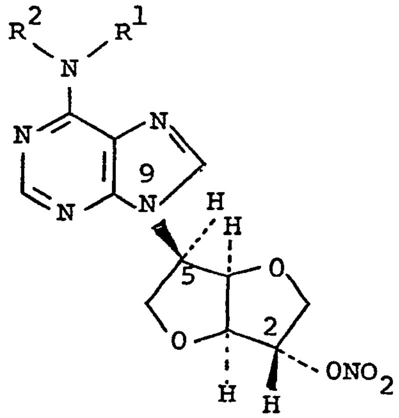

(Ie)

wherein $R^1$ and $R^2$ possess the meanings mentioned in claim 1, as well as their physiologically acceptable acid-addition salts.

6. 5-(7-Theophyllinyl)-5-desoxy-1.4;3.6-dianhydro-L-iditol 2-nitrate, as well as its physiologically acceptable acid-addition salts.

7. 2-(7-Theophyllinyl)-2-desoxy-1.4;3.6-dianhydro-D-glucitol 5-nitrate, as well as its physiologically acceptable acid addition salts.

8. 5-(9-Adenyl)-5-desoxy-1.4;3.6-dianhydro-L-iditol 2-nitrate.

9. 5-(6-Methylaminopurin-9-yl)-5-desoxy-1.4;3.6-dianhydro-L-iditol 2-nitrate.

10. 5-(6-Dimethylaminopurin-9-yl)-5-desoxy-1.4;3.6-dianhydro-L-iditol 2-nitrate.

11. 5-(6-Ethylaminopurin-9-yl)-5-desoxy-1.4;3.6-dianhydro-L-iditol 2-nitrate.

12. 5-(6-Pyrrolidinopurin-9-yl)-5-desoxy-1.4;3.6-dianhydro-L-iditol 2-nitrate.

13. 5-(6-Piperidinopurin-9-yl)-5-desoxy-1.4;3.6-dianhydro-L-iditol 2-nitrate.

14. 5-(6-Morpholinopurin-9-yl)-5-desoxy-1.4;3.6-dianhydro-L-iditol 2-nitrate.

15. 5-[6-(4-Methylpiperazino)-purin-9-yl]-5-desoxy-1.4;3.6-dianhydro-L-iditol 2-nitrate.

16. 5-[9-(6-N-Pivaloyl)-adenyl]-5-desoxy-1.4;3.6-dianhydro-L-iditol 2-nitrate.

17. 5-[9-(6-N-Acetyl)-adenyl]-5-desoxy-1.4;3.6-dianhydro-L-iditol 2-nitrate.

18. 5-(6-Benzylaminopurin-9-yl)-5-desoxy-1.4;3.6-dianhydro-L-iditol 2-nitrate.

19. 5-[6-(3-Phenylpropyl)-aminopurin-9-yl]-5-desoxy-1.4;3.6-dianhydro-L-iditol 2-nitrate.

20. 2-(9-Adenyl)-2-desoxy-1.4;3.6-dianhydro-D-glucitol 5-nitrate.

21. 5-{6-[3-(4-Chlorophenyl)-propylamino]-purin-9-yl}-5-desoxy-1.4;3.6-dianhydro-L-iditol 2-nitrate.

22. Process for the preparation of the compounds according to claim 1, characterised in that

(A) L-isoidide, D-Isosorbide or D-isomannide is converted with a sulphonic acid chloride into the corresponding mono-O-acyl-1.4;3.6-dianhydrohexitol,

(B) which is reacted with an alkali metal salt of adenine or of an adenine substituted in the 6-N-position, of 6-methylmercaptopurine or 6-chloropurine, with reversal of the configuration at the point of substitution, to give the corresponding monopurinyl-1.4;3.6-dianhydrohexitol,

(C) whereafter the 6-methylmercapto group or the 6-chlorine atom of the adenine residue is possibly reacted with a primary or secondary alkylamine with straight-chained or branched alkyl group with 1 to 7 C-atoms or with an ω-phenylalkylamine with an alkyl group with 1 to 7 C-atoms or with a corresponding p-halogen-substituted ω-phenylalkylamine or with a cyclic, non-aromatic secondary amine possibly containing a further hetero atom,

(D) whereafter the free hydroxyl group of the resulting adenyldesoxy-1.4;3.6-dianhydrohexitol is esterified in per se known manner with nitric acid, nitrating acid or with a mixture of nitric acid and glacial acetic acid/acetic anhydride to give the corresponding mononitrates of the general formulae Ia and Ic to Ie,

(E) whereafter the 6-amino group or the monosubstituted 6-amino group of the adenine residue is possibly reacted in per se known manner with an aliphatic, possibly methyl-substituted monocarboxylic acid with 2 to 7 C-atoms to give the 6-acylamino group.

23. Process for the preparation of compounds according to claim 3, characterised in that

(A) D-isosorbide is reacted quantitatively with an excess of sulphonic acid chloride, preferably methanesulphonyl chloride or toluenesulphonyl chloride, to give the corresponding isosorbide diacylate,

(B) which is reacted with an alkali metal salt of adenine or of an adenine substituted in the 6-N-position to give the corresponding 5-(9-adenyl)-5-desoxy-1.4;3.6-dianhydro-L-iditol 2-acylate,

(C) whereafter the 2-acylate group is subjected to an acidic or alkaline hydrolysis and

(D) the resultant 5-(9-adenyl)-5-desoxy-1.4;3.6-dianhydro-L-iditol is further worked up corresponding to the process according to claim 22 (D) + (E).

24. Process for the preparation of compounds according to claim 2, characterised in that one works

according to claim 22, but, instead of an alkali metal salt of adenine, of a 6-N-substituted adenine, of 6-methylmercaptopurine or of 6-chloropurine, there is used an alkali metal salt of theophyllin.

25. Process for the preparation of the compounds according to claim 6, characterised in that one works according to claim 23, but, instead of an alkali metal salt of adenine or of a 6-N-substituted adenine, there is used an alkali metal salt of theophyllin.

26. Process according to one of claims 22 or 24, characterised in that the reaction of the D-isosorbide 5-acylate or of the D-isomannide 2-acylate with an alkali metal salt of adenine, of an adenine substituted in the 6-N-position, of 6-methylmercaptopurine, of 6-chloropurine or of theophyllin takes place in a dipolar aprotic solvent at a temperature between 80 and 130 °C under an inert gas atmosphere.

27. Process according to claim 22, characterised in that the reaction of the 6-methylmercapto group or of the 6-chloro atom of the purinyl residue with ammonia or a primary or secondary amine takes place at a temperature of 100 to 170 °C and at a pressure of 2 to 20 ats. under an inert gas atmosphere.

28. Process according to one of claims 23 or 25, characterised in that the reaction of the D-isosorbide diacylate with an alkali metal salt of adenine, of an adenine substituted in the 6-N-position or of theophyllin takes place in a dipolar aprotic solvent at a temperature between 80 and 110 °C under an inert gas atmosphere.

29. Process according to claim 26 or 28, characterised in that dimethylformamide or dimethylsulphoxide is used as said dipolar aprotic solvent.

30. Process according to claim 27, characterised in that the reaction takes place in the presence of a lower alcohol as a solvent.

31. Pharmaceutical composition, containing a compound according to one of claims 1 to 21, as well as conventional carriers and additive materials.

**Revendications**

1. Adényl-désoxy-1.4;3.6-dianhydro-hexite-nitrates de formule générale Ia

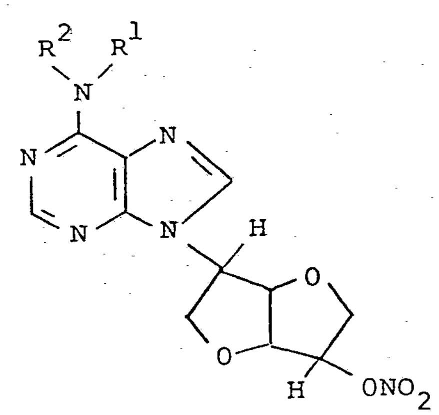

(Ia)

où $R^1$ et $R^2$ sont semblables ou différents et représentent indépendamment l'un de l'autre

(a) un atome d'hydrogène,

(b) un groupe alcoyle en $C_1$ à $C_7$ à chaîne droite ou ramifiée,

(c) un groupe ω-phénylalcoyle, où le groupe alcoyle présente de 1 à 7 atomes de carbone et où le noyau phényle peut être halogénosubstitué en position p,

ou bien ou

(d) $R^1$ représente l'un des radicaux indiqués en (a) à (c) et $R^2$ représente un radical acyle d'un acide monocarboxylique en $C_2$ à $C_7$ aliphatique, éventuellement méthyl-substitué,

ou bien où

(e) $R^1$ et $R^2$ représentent ensemble avec l'atome d'azote auquel ils sont liés le radical d'une amine secondaire cyclique, non aromatique, contenant éventuellement un autre hétéroatome, qui est choisie dans le groupe constitué par la pyrrolidine, la pipéridine, la pipérazine, la 4-méthylpipérazine et la morpholine,

ainsi que leurs sels d'addition d'acides physiologiquement acceptables.

2. Théophyllinyl-désoxy-1.4;3.6-dianhydro-hexite-nitrates de formule générale Ib,

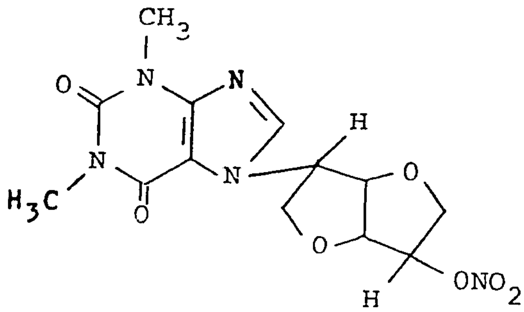

(Ib)

ainsi que leurs sels d'addition d'acides physiologiquement acceptables.

3. 5-(9-adényl)-5-désoxy-1.4;3.6-dianhydro-L-idite-2-nitrates de formule générale Ic,

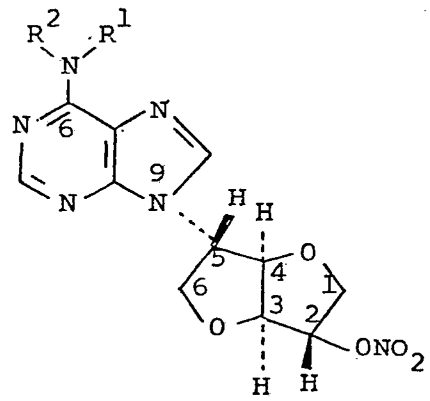

(Ic)

où $R^1$ et $R^2$ ont les significations données dans la revendication 1, ainsi que leurs sels d'addition d'acides physiologiquement acceptables.

4. 2-(9-adényl)-2-désoxy-1.4;3.6-dianhydro-D-glucite-5-nitrates de formule générale Id,

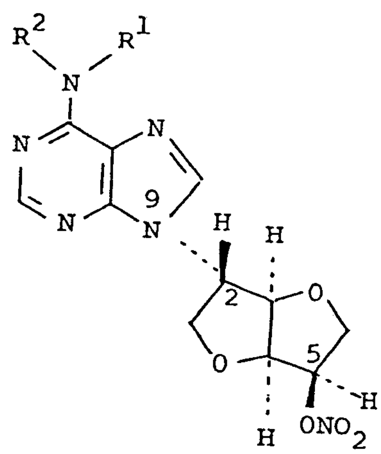

(Id)

où $R^1$ et $R^2$ ont les significations données dans la revendication 1, ainsi que leurs sels d'addition d'acides physiologiquement acceptables.

5. 5-(9-adényl)-5-désoxy-1.4;3.6-dianhydro-D-glucite-2-nitrates de formule générale Ie,

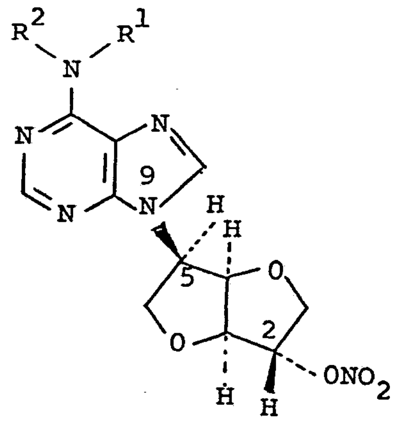

(Ie)

où $R^1$ et $R^2$ ont les significations données dans la revendication 1, ainsi que leurs sels d'addition d'acides physiologiquement acceptables.

6. 5-(7-théophyllinyl)-5-désoxy-1.4;3.6-dianhydro-L-idite-2-nitrate ainsi que ses sels d'addition d'acides physiologiquement acceptables.

7. 2-(7-théophyllinyl)-2-désoxy-1.4;3.6-dianhydro-D-glucite-5-nitrate ainsi que ses sels d'addition d'acides physiologiquement acceptables.

8. 5-(9-adényl)-5-désoxy-1.4;3.6-dianhydro-L-idite-2-nitrate.

9. 5-(6-méthylaminopurin-9-yl)-5-désoxy-1.4;3.6-dianhydro-L-idite-2-nitrate.

10. 5-(6-diméthylaminopurin-9-yl)-5-désoxy-1.4;3.6-dianhydro-L-idite-2-nitrate.

11. 5-(6-éthylaminopurin-9-yl)-5-désoxy-1.4;3.6-dianhydro-L-idite-2-nitrate.

12. 5-(6-pyrrolidinopurin-9-yl)-5-désoxy-1.4;3.6-dianhydro-L-idite-2-nitrate.

13. 5-(6-pipéridinopurin-9-yl)-5-désoxy-1.4;3.6-dianhydro-L-idite-2-nitrate.

14. 5-(6-morpholinopurin-9-yl)-5-désoxy-1.4;3.6-dianhydro-L-idite-2-nitrate.

15. 5-[6-(4-méthylpipérazino)-purin-9-yl]-5-désoxy-1.4;3.6-dianhydro-L-idite-2-nitrate.

16. 5-[9-(6-N-pivaloyl)-adényl]-5-désoxy-1.4;3.6-dianhydro-L-idite-2-nitrate.

17. 5-[9-(6-N-acétyl)-adényl]-5-désoxy-1.4;3.6-dianhydro-L-idite-2-nitrate.

18. 5-(6-benzylaminopurin-9-yl)-5-désoxy-1.4;3.6-dianhydro-L-idite-2-nitrate.

19. 5-[6-(3-phénylpropyl)-aminopurin-9-yl]-5-désoxy-1.4;3.6-dianhydro-L-idite-2-nitrate.

20. 2-(9-adényl)-2-désoxy-1.4;3.6-dianhydro-D-glucite-5-nitrate.

21. 5-{6-[3-(4-chlorophényl)-propylamino]-purin-9-yl}-5-désoxy-1.4;3.6-dianhydro-L-idite-2-nitrate.

22. Procédé de préparation des composés selon la revendication 1, caractérisé en ce que

(A) on transforme le L-isoidide, le D-isosorbide ou le D-isomannide avec un chlorure d'acide sulfonique en le mono-O-acyl-1.4;3.6-dianhydro-hexite correspondant,

(B) que l'on fait réagir avec un sel alcalin de l'adénine ou d'une adénine substituée en position 6-N, de la 6-méthylmercaptopurine ou de la 6-chloropurine avec inversion de la configuration à l'endroit de la substitution pour donner le mono-purinyl-1.4;3.6-dianhydro-hexite correspondant,

(C) après quoi on fait éventuellement réagir le groupe 6-méthylmercapto ou l'atome de chlore 6 du radical adénine avec une alcoylamine primaire ou secondaire à groupe alcoyle à chaîne droite ou ramifiée en $C_1$ à $C_7$ ou avec une ω-phénylalcoylamine avec un groupe alcoyle en $C_1$ à $C_7$ ou avec une ω-phénylalcoylamine p-halogénosubstituée correspondante ou avec une amine secondaire cyclique, non aromatique, contenant éventuellement un autre hétéroatome,

(D) après quoi on estérifie le groupe hydroxyle libre de l'adényl-désoxy-1.4;3.6-dianhydro-hexite apparu de façon connue avec de l'acide nitrique, du mélange sulfonitrique ou avec un mélange d'acide nitrique et d'acide acétique glacial/acétanhydride pour donner les mononitrates correspondants de formules générales Ia et Ic à Ie

(E) après quoi on fait éventuellement réagir le groupe 6-amino ou le groupe 6-amino monosubstitué du radical adénine de façon connue avec un acide monocarboxylique aliphatique en $C_2$ à $C_7$ éventuellement méthyl-substitué pour donner le groupe 6-acylamino.

23. Procédé de préparation des composés selon la revendication 3, caractérisé en ce que

(A) on fait réagir le D-isosorbide avec un excès de chlorure d'acide sulfonique, de préférence le chlorure de méthanesulfonyle ou le chlorure de toluènesulfonyle, de façon quantitative pour donner le diacylate d'isosorbide correspondant,

(B) que l'on fait réagir avec un sel alcalin de l'adénine ou d'une adénine substituée en position 6-N pour donner le 5-(9-adényl)-5-désoxy-1.4;3.6-dianhydro-L-idite-2-acylate correspondant,

(C) après quoi on soumet le groupe 2-acylate à une hydrolyse acide ou alcaline et

(D) on traite plus avant le 5-(9-adényl)-5-désoxy-1.4;3.6-dianhydro-L-idite apparu de façon correspondant au procédé selon la revendication 22 (D) + (E).

24. Procédé de préparation des composés selon la revendication 2, caractérisé en ce qu'on travaille

selon la revendication 22, mais en ce qu'on utilise à la place d'une adénine 6-N-substituée, de la 6-méthylmercaptopurine ou de la 6-chloropurine un sel alcalin de la théophylline.

25. Procédé de préparation des composés selon la revendication 6, caractérisé en ce qu'on travaille selon la revendication 23, mais en ce qu'on utilise à la place d'un sel alcalin de l'adénine ou d'une adénine 6-N-substituée un sel alcalin de la théophylline.

26. Procédé selon l'une des revendications 22 ou 24, caractérisé en ce que la réaction du D-isosorbide-5-acylate ou selon les cas du D-isomannide-2-acylate avec un sel alcalin de l'adénine, d'une adénine substituée en position 6-N, de la 6-méthylmercaptopurine, de la 6-chloropurine ou de la théophylline s'effectue dans un solvant aprotique dipolaire à une température comprise entre 80 et 130 °C sous une atmosphère de gaz inerte.

27. Procédé selon la revendication 22, caractérisé en ce que la réaction du groupe 6-méthylmercapto ou de l'atome de chlore en position 6 du radical purinyle s'effectue avec de l'ammoniac ou une amine primaire ou secondaire à une température de 100 à 170 °C et à une pression de 2 à 20 atm sous une atmosphère de gaz inerte.

28. Procédé selon l'une des revendications 23 ou 25, caractérisé en ce que la réaction du D-isosorbide-diacylate avec un sel alcalin de l'adénine, d'une adénine substituée en position 6-N ou de la théophylline s'effectue dans un solvant aprotique dipolaire a une température comprise entre 80 et 110 °C sous une atmosphère de gaz inerte.

29. Procédé selon les revendications 26 ou 28, caractérisé en ce qu'on utilise le diméthylformamide ou le diméthylsulfoxyde comme solvant aprotique dipolaire.

30. Procédé selon la revendication 27, caractérisé en ce que la réaction s'effectue en présence d'un alcool inférieur comme solvant.

31. Préparation pharmaceutique contenant un composé selon l'une des revendications 1 à 21 ainsi que les supports et additifs habituels.